# EUROPEAN PATENT SPECIFICATION

(11) **EP 3 385 258 B1**
(45) Date of publication and mention of the grant of the patent: **24.04.2024**
(21) Application number: 18172100.2
(22) Date of filing: 10.05.2012
(51) Int. Cl.: C07D 307/68

(54) **AN OXIDATION PROCESS TO PRODUCE A CRUDE AND/OR PURIFIED FURAN-DI-CARBOXYLIC ACID PRODUCT**
OXIDATIONSVERFAHREN ZUR HERSTELLUNG EINES ROHEN UND/ODER GEREINIGTEN FURAN-DI-CARBONSÄUREPRODUKTS
PROCÉDÉ D'OXYDATION PERMETTANT LA PRODUCTION D'UN PRODUIT DE TYPE FURANE-ACIDE DI-CARBOXYLIQUE BRUT ET/OU PURIFIÉ

(30) Priority: 24.05.2011 US 201161489496 P; 09.09.2011 US 201113228816
(43) Date of publication of application: 10.10.2018
(62) Divisional of application: 12789055.6
(73) Proprietor: Eastman Chemical Company, Kingsport, TN 37660 (US)
(72) Inventor: Janka, Mesfin Ejerssa, Kingsport, TN 37660 (US); Lange, David Milton, Blountville, TN 37617 (US); Morrow, Michael Charles, Kingsport, TN 37664 (US); Bowers, Bradford Russell, Chuckey, TN 37641 (US); Parker, Kenny Randolph, Afton, TN 37616 (US); Shaikh, Ashfaq, Kingsport, TN 37660 (US); Partin, Lee Reynolds, Lexington, KY 40511 (US); Sumner, Charles Edwan Jr., Kingsport, TN 37664 (US)
(74) Representative: Ricker, Mathias

(56) References cited:
- WO-A2-01/72732
- WO-A2-2012/161970
- GB-A- 934 292
- KROEGER M ET AL: "A new approach for the production of 2,5-furandicarboxylic acid by in situ oxidation of 5-hydroxymethylfurfural starting from fructose", TOPICS IN CATALYSIS, BALTZER SCIENCE PUBLISHERS, BUSSUM, NL, vol. 13, 1 January 2000 (2000-01-01), pages 237-242, XP002471377, ISSN: 1022-5528, DOI: 10.1023/A:1009017929727
- J. KISS ET AL: "�ber Muscarin. 11. Mitteilung. Synthese von bisquatern�ren, dem Muscarin �hnlichen Verbindungen", HELVETICA CHIMICA ACTA, vol. 44, no. 1, 1 January 1961 (1961-01-01), pages 141-147, XP055090528, ISSN: 0018-019X, DOI: 10.1002/hlca.19610440119
- VERDEGUER P ET AL: "Oxydation catalytique du HMF en acide 2,5-furane dicarboxylique", JOURNAL OF MOLECULAR CATALYSIS, ELSEVIER, NL, vol. 85, 1 January 1993 (1993-01-01), pages 327-344, XP002471376, ISSN: 0304-5102, DOI: 10.1016/0304-5102(93)80059-4

## Description

### FIELD OF THE INVENTION

The present invention relates to a purified carboxylic acid composition produced by a process comprising oxidizing 5-hydroxylmethyl)furfural in the presence of oxygen, a saturated organic acid solvent having from 2-6 carbon atoms, and a catalyst system comprising cobalt, manganese and bromine, wherein the source of bromine includes hydrobromic acid, at a temperature of 100°C to 220°C to produce the carboxylic acid composition comprising furan-2,5-dicarboxylic acid, as defined in claim 1.

### BACKGROUND OF THE INVENTION

Aromatic dicarboxylic acids, such as terephthalic acid and isophthalic acid, are used to produce a variety of polyester products. Important examples of which are poly (ethylene terephthalate) and its copolymers. These aromatic dicarboxylic acids are synthesized by the catalytic oxidation of the corresponding dialkyl aromatic compounds which are obtained from fossil fuels, which is disclosed in U.S. Patent Application 2006/0205977 A1.

There is a growing interest in the use of renewable resources as feed stocks for the chemical industry mainly due to the progressive reduction of fossil reserves and their related environmental impacts. Furan-2,5-dicarboxylic acid (FDCA) is a versatile intermediate considered as a promising closest biobased alternative to terephthalic acid and isophthalic acid. Like aromatic diacids, FDCA can be condensed with diols such as ethylene glycol to make polyester resins similar to polyethylene terephthalate (PET) (Gandini, A.; Silvestre, A. J; Neto, C. P.; Sousa, A. F.; Gomes, M. J. Poly. Sci. A 2009, 47, 295.). Therefore, there is a need in the chemical industry for an efficient process to produce carboxylic acid compositions, especially FDCA. A high yield process (minimum of 90% FDCA yield) to produce a dry, purified FDCA product is provided herein.

P. Vereguer et al., "Oxidation catalytique du HMF en acide 2,5-difuran dicarboxylique", J. Mol. Catal. 85 (1923) 327-344, disclose the catalytic oxidation of hydroxymethyl furfural to 2,5-furandicarboxylic acid, wherein the reaction is carried out in a strongly alkaline aqueous medium and in the presence of oxygen and a platinum/lead catalyst deposited on carbon.

WO 2001072732 discloses the oxidation of hydroxymethyl furfural in acetic acid in the presence of oxygen and catalyst metals based on Co/Mn/Br.

### BRIEF DESCRIPTION OF THE DRAWINGS

Figure 1 illustrates different embodiments of the invention wherein a process to produce a dried purified carboxylic acid **710** is provided.
Figure 2 illustrates an embodiment of the invention, showing the GC chromatogram of the carboxylic acid composition **110** that has been dried.
Figure 3 illustrates an embodiment of the invention, showing a ¹H NMR of the carboxylic acid composition **110** that has been dried.
Figure 4 illustrates an embodiment of the invention, showing a ¹³C{¹H} NMR of the carboxylic acid composition **110** that has been dried.
Figure 5 illustrates an embodiment of the invention, showing the effects of temperature, pressure, cobalt and bromine concentrations have on the FDCA yield in the carboxylic acid composition **110.**
Figure 6 illustrates an embodiment not belonging to the invention, Effect of temperature on yield using an oxidizable raw material stream **30** comprising 5-AMF. The + symbol represents that Co is 2000 ppm and Br is 3000 ppm. The x symbol represents that Co is 2500 ppm and Br is 2500 ppm.
Figure 7 illustrates an embodiment not belonging to the invention, Effect of temperature on yield using an oxidizable raw material stream **30** comprising 5-EMF. The + symbol represents that Co is 2000 ppm and Br is 3000 ppm. The x symbol represents that Co is 2500 ppm and Br is 2500 ppm.

### DETAILED DESCRIPTION

It should be understood that the following is not intended to be an exclusive list of defined terms. Other definitions may be provided in the foregoing description, such as, for example, when accompanying the use of a defined term in context.

As used herein, the terms "a," "an," and "the" mean one or more.

As used herein, the term "and/or," when used in a list of two or more items, means that any one of the listed items can be employed by itself or any combination of two or more of the listed items can be employed. For example, if a composition is described as containing components A, B, and/or C, the composition can contain A alone; B alone; C alone; A and B in combination; A and C in combination, B and C in combination; or A, B, and C in combination.

As used herein, the terms "comprising," "comprises," and "comprise" are open-ended transition terms used to transition from a subject recited before the term to one or more elements recited after the term, where the element or elements listed after the transition term are not necessarily the only elements that make up the subject.

As used herein, the terms "having," "has," and "have" have the same open-ended meaning as "comprising," "comprises," and "comprise" provided above.

As used herein, the terms "including," "includes," and "include" have the same open-ended meaning as "comprising," "comprises," and "comprise" provided above.

The present description uses numerical ranges to quantify certain parameters relating to the invention. It should be understood that when numerical ranges are provided, such ranges are to be construed as providing literal support for claim limitations that only recite the lower value of the range as well as claim limitations that only recite the upper value of the range. For example, a disclosed numerical range of 10 to 100 provides literal support for a claim reciting "greater than 10" (with no upper bounds) and a claim reciting "less than 100" (with no lower bounds).

The present description uses specific numerical values to quantify certain parameters relating to the invention, where the specific numerical values are not expressly part of a numerical range. It should be understood that each specific numerical value provided herein is to be construed as providing literal support for a broad, intermediate, and narrow range. The broad range associated with each specific numerical value is the numerical value plus and minus 60 percent of the numerical value, rounded to two significant digits. The intermediate range associated with each specific numerical value is the numerical value plus and minus 30 percent of the numerical value, rounded to two significant digits. The narrow range associated with each specific numerical value is the numerical value plus and minus 15 percent of the numerical value, rounded to two significant digits. For example, if the specification describes a specific temperature of 17 °C (62 °F), such a description provides literal support for a broad numerical range of - 4 °C to 37 °C (16.5 +/- 20.5) [25 °F to 99 °F (62 °F +/- 37 °F)], an intermediate numerical range of 6 °C to 27 °C (16.5 °C +/- 10.5 °C) [43 °F to 81 °F (62 °F +/- 19 °F)], and a narrow numerical range of 11 °C to 22 °C (16.5 °C +/- 5.5 °C) [53 °F to 71 °F (62 °F +/- 9 °F)]. These broad, intermediate, and narrow numerical ranges should be applied not only to the specific values, but should also be applied to differences between these specific values. Thus, if the specification describes a first pressure of 7.7 bar (110 psia) and a second pressure of 3.4 bar (48 psia) (a difference of 4.3 bar (62 psi)), the broad, intermediate, and narrow ranges for the pressure difference between these two streams would be 17.5 bar to 6.9 bar (25 to 99 psi), 3.0 bar to 5.7 bar (43 to 81 psi), and 3.7 bar to 5 bar (53 to 71 psi), respectively

The invention relates to a purified carboxylic composition as defined in claim 1. Preferred embodiments are defined in the claims depending thereon.

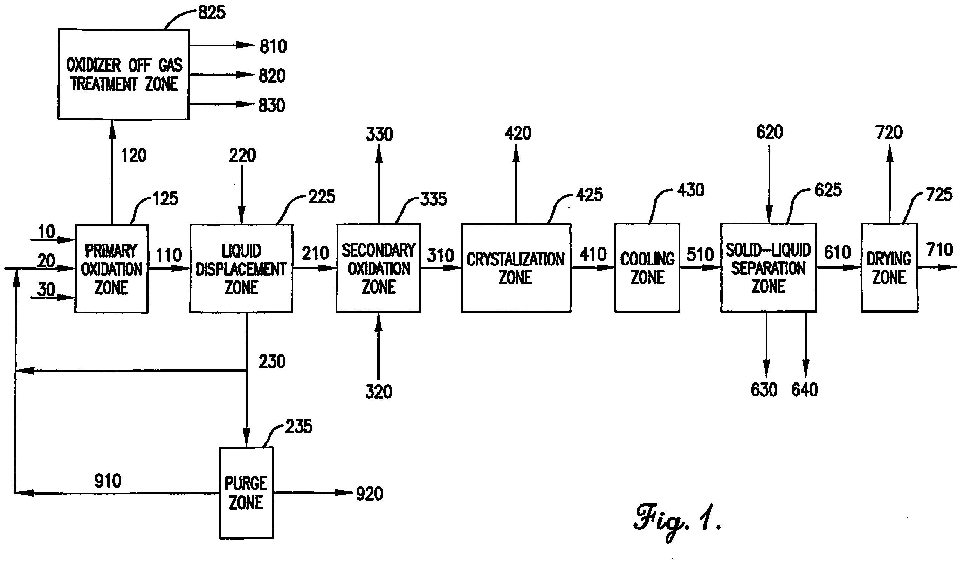

Disclosed is a process to produce a carboxylic acid composition and/or dry purified carboxylic acid 710 comprising furan-2,5-dicarboxylic acid (FDCA). Embodiments of the process are represented in **Figure 1****.** The process comprises oxidizing at least one oxidizable compound in an oxidizable raw material stream 30 in the presence of an oxidizing gas stream **10**, solvent stream **20**, and at least one catalyst system. The oxidizable raw material stream **30** comprises at least one oxidizable compound suitable to produce a carboxylic acid composition **110** comprising FDCA. The amount of FDCA in the carboxylic acid composition **110** can range from greater than 10 by weight percent in the carboxylic acid composition **110**, greater than 20 by weight percent in the carboxylic acid composition **110**, greater than 30 by weight percent in the carboxylic acid composition **110.** The carboxylic acid composition **110** comprises FDCA and solvent.

In another embodiment, the process comprises oxidizing at least one oxidizable compound in an oxidizable raw material stream **30** in the presence of an oxidizing gas stream **10**, solvent stream **20**, and at least one catalyst system. The oxidizable raw material stream **30** comprises 5-(hydroxymethyl)furfural (5-HMF), mixed feedstocks of 5-HMF and 5-HMF esters (5-R(CO)OCH₂-furfural where R = alkyl, cycloalkyl and aryl), mixed feedstocks of 5-HMF and 5-HMF ethers (5-R'OCH₂-furfural, where R' = alkyl, cycloalkyl and aryl), and mixed feedstocks of 5-HMF and 5-alkyl furfurals (5-R"-furfural, where R" = alkyl, cycloalkyl and aryl) to generate a carboxylic acid composition comprising FDCA. The process includes removing impurities from the carboxylic acid composition **110** in a liquid displacement zone **225** to form a low impurity slurry stream **210.** The low impurity slurry stream **210** can be further treated in a secondary oxidation zone **335** to produce a secondary oxidation slurry stream **310** which can be routed to a crystallization zone **425** to form a crystallized slurry stream **410.** The crystallized slurry stream **410** is cooled in a cooling zone **430** and the cooled crystallized slurry stream **510** can be routed to a solid-liquid separation zone **625** to generate a purified wet cake stream **610** comprising FDCA that is dried in a drying zone **725** to generate a dried, purified carboxylic acid **710** comprising purified FDCA.

In one embodiment, a process is provided to produce a dried, purified carboxylic acid **710** comprising dried, purified furan-2,5-dicarboxylic acid (FDCA) and comprises the following steps:
**Step (a)** comprises oxidizing at least one oxidizable compound in an oxidizable raw material stream **30** in the presence of an oxidizing gas stream **10**, solvent stream **20**, and at least one catalyst system in a primary oxidation zone **125** which comprises at least one primary oxidizer reactor to produce a carboxylic acid composition **110** comprising furan-2,5-dicarboxylic(FDCA); wherein the oxidizable raw material stream **30** comprises at least one oxidizable compound selected from the group consisting of 5-(hydroxymethyl)furfural (5-HMF), mixed feedstocks of 5-HMF and 5-HMF esters (5-R(CO)OCH₂-furfural where R = alkyl, cycloalkyl and aryl), mixed feedstocks of 5-HMF and 5-HMF ethers (5-R'OCH₂-furfural, where R' = alkyl, cycloalkyl and aryl), and mixed feedstocks of 5-HMF and 5-alkyl furfurals (5-R"-furfural, where R" = alkyl, cycloalkyl and aryl). Structures for the various oxidizable raw material compounds are outlined below:
Preferred 5-HMF Derivative Feeds

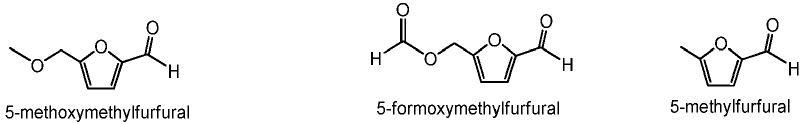

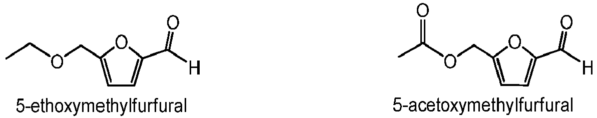

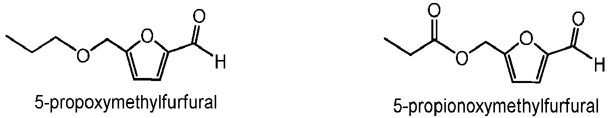

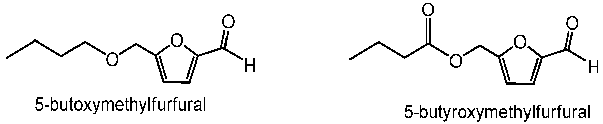

The 5-HMF or said mixed feedstocks of 5-HMF and 5-HMF esters, 5-HMF and 5-HMF ethers, or 5-HMF and 5-alkyl furfurals are oxidized with elemental O₂ in a multi-step reactions, eqs 1 and 2, to form FDCA with 5-formyl furan-2-carboxyic acid (FFCA) as a key intermediate.

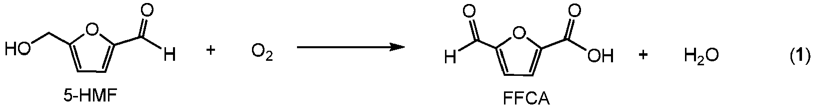

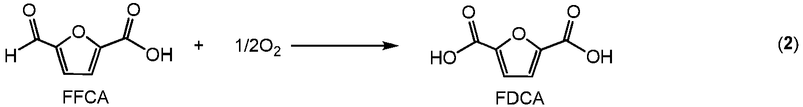

In one embodiment, streams routed to the primary oxidation zone **125** comprises an oxidizing gas stream **10** comprising oxygen and a solvent stream **20** comprising solvent, an oxidizable raw material stream **30**, and a catalyst system. Oxidizable raw material stream 30 comprises a continuous liquid phase. In another embodiment of the invention, the oxidizable raw material stream **30**, the oxidizing gas stream **10**, the solvent stream **20** and the catalyst system can be fed to the primary oxidization zone **125** as separate and individual streams or combined in any combination prior to entering the primary oxidation zone **125** wherein said feed streams may enter at a single location or in multiple locations in the primary oxidization zone **125.**

The carboxylic acid composition **110** comprises FDCA and FFCA, wherein the FFCA in the carboxylic acid composition **110** ranges from 0.1 wt% (weight percent) to 4 wt% or 0.1 wt% to 0.5 wt%, or 0.1wt% to 1wt%. In another embodiment of the invention the carboxylic acid composition **110** comprises FDCA and FFCA and at least one of 2,5-diformylfuran in an amount ranging from 0 wt% to 0.2 wt%, levulinic acid in an amount ranging from 0 wt% to 0.5 wt%, succinic acid in an amount ranging from 0 wt% to 0.5 wt% and acetoxy acetic acid in an amount ranging from 0 wt% to 0.5 wt%.

In another embodiment of the invention the carboxylic acid composition **110** comprises FDCA, FFCA and EFCA. In other embodiment of the invention the EFCA in the carboxylic acid composition **110** in an range from 0.05 wt% to 4 wt%, or 1 wt% to 2 wt%.

The catalyst system comprises at least one catalyst suitable for oxidation comprising cobalt, bromine and manganese compounds as defined in claim 1, which are soluble in the selected oxidation solvent. In one embodiment of the invention, the catalyst system comprises cobalt, manganese and bromine wherein the weight ratio of cobalt to manganese in the reaction mixture is from about 10 to about 400 and the weight ratio of cobalt to bromine is from about 0.7 to about 3.5.

The oxidizing gas stream comprises oxygen. Examples include, but are not limited to, air and purified oxygen. The amount of oxygen in the primary oxidation zone ranges from 5 mole % to 45 mole %, 5 mole % to 60 mole % 5 mole % to 80 mole %.

Suitable solvents include water and the aliphatic solvents. In an embodiment of the invention, the solvents are aliphatic carboxylic acids which include, but are not limited to, aqueous solutions of C₂ to C₆ monocarboxylic acids, e.g., acetic acid, propionic acid, n-butyric acid, isobutyric acid, n-valeric acid, trimethylacetic acid, caprioic acid, and mixtures thereof.

The most common solvent used for the oxidation is an aqueous acetic acid solution, typically having a concentration of 80 to 99 wt. %. In especially preferred embodiments, the solvent comprises a mixture of water and acetic acid which has a water content of 0% to 15% by weight. Additionally, a portion of the solvent feed to the primary oxidation reactor may be obtained from a recycle stream obtained by displacing 80 to 90% of the mother liquor taken from the crude reaction mixture stream discharged from the primary oxidation reactor with fresh, wet acetic acid containing 0 to 15% water.

Suitable solvents comprise mixtures of aliphatic mono-carboxylic acids containing 2 to 6 carbon atoms and water. Examples of aliphatic mono-carboxylic acids, include, but are not limited to acetic acid.

Generally, the oxidation temperature varies from 100°C to 220°C and from 110°C to 160°C.

The process is provided to produce furan-2,5-dicarboxylic acid (FDCA) in high yields by liquid phase oxidation that minimizes solvent and starting material loss through carbon burn. The process comprises oxidizing at least one oxidizable compound in an oxidizable raw material stream **30** in the presence of an oxidizing gas stream **10**, solvent stream **20**, and at least one catalyst system in a primary oxidation zone **125**; wherein the oxidizable compound is 5-(hydroxymethyl)furfural (5-HMF). The oxidizable compound can be oxidized in a solvent comprising acetic acid with or without the presence of water with oxygen in the presence of a catalyst system comprising cobalt, manganese and bromine, wherein the weight ratio of cobalt to manganese in the reaction mixture is from about 10 to about 400 and the weight ratio of cobalt to bromine is from about 0.7 to about 3.5. Such a catalyst system with improved Co:Mn ratio can lead to high yield of FDCA. In this process, the oxidation temperature varies from 100°C to 220°C, or another range from 110°C to 160°C, which can minimize carbon burn. The cobalt concentration of the catalyst can range from 1000 ppm to 6000 ppm, and the amount of manganese ranges from 2 ppm to 600 ppm, and the amount of bromine ranges from 300 ppm to 4500 ppm with respect to the total weight of the liquid in the reaction medium of the primary oxidation zone **125.** As used herein, process temperature is the temperature of the reaction mixture within the primary oxidation zone where liquid is present as the continuous phase. The primary oxidizer reactor will typically be characterized by a lower section where gas bubbles are dispersed in a continuous liquid phase. Solids can also be present in the lower section. In the upper section of the primary oxidizer, gas is in the continuous phase and entrained liquid drops can also be present.

The catalyst compositions employed in the process of the invention comprise cobalt atoms, manganese atoms, and bromine atoms, supplied by any suitable means, as further described below. The catalyst composition is typically soluble in the solvent under reaction conditions, or it is soluble in the reactants fed to the oxidation zone. Preferably, the catalyst composition is soluble in the solvent at 40° C. and 0.96 bar (1 atm), and is soluble in the solvent under the reaction conditions.

The cobalt atoms may be provided in ionic form as inorganic cobalt salts, such as cobalt bromide, cobalt nitrate, or cobalt chloride, or organic cobalt compounds such as cobalt salts of aliphatic or aromatic acids having 2-22 carbon atoms, including cobalt acetate, cobalt octanoate, cobalt benzoate, cobalt acetylacetonate, and cobalt naphthalate.

The oxidation state of cobalt when added as a compound to the reaction mixture is not limited, and includes both the +2 and +3 oxidation states.

The manganese atoms may be provided as one or more inorganic manganese salts, such as manganese borates, manganese halides, manganese nitrates, or organometallic manganese compounds such as the manganese salts of lower aliphatic carboxylic acids, including manganese acetate, and manganese salts of beta-diketonates, including manganese acetylacetonate.

According to the invention, suitable sources of bromine include hydrobromic acid.

In another embodiment, a process is provided for producing furan-2,5-dicarboxylic acid (FDCA) in high yields by liquid phase oxidation that minimizes solvent and starting material loss through carbon burn. The process comprises oxidizing at least one oxidizable compound in an oxidizable raw material stream **30** in the presence of an oxidizing gas stream **10**, solvent stream **20**, and at least one catalyst system in a primary oxidation zone **125;** wherein said oxidizable compound is 5-(hydroxymethyl)furfural (5-HMF); wherein said solvent stream comprises acetic acid with or without the presence of water; wherein said catalyst system comprising cobalt, manganese and bromine, wherein the weight ratio of cobalt to manganese in the reaction mixture is from 10 to 400. In this process, the temperature varies from 100°C to 220°C, from 105°C to 180°C, and from 110°C to 160°C. The cobalt concentration of the catalyst system can range from 1000 ppm to 6000 ppm, and the amount of manganese ranges from 2 ppm to 600 ppm, and the amount of bromine ranges from 300 ppm to 4500 ppm with respect to the total weight of the liquid in the reaction medium.

The process comprises oxidizing at least one oxidizable compound in an oxidizable raw material stream **30** in the presence of an oxidizing gas stream **10**, solvent stream **20**, and at least one catalyst system in a primary oxidation zone **125;** wherein said oxidizable compound is 5-(hydroxymethyl)furfural (5-HMF); wherein said solvent stream comprises a saturated organic acid having from 2-6 carbon atoms with or without the presence of water at a temperature of 100°C to 220°C to produce a dicarboxylic acid composition; wherein the primary oxidation zone **125** comprises at least one primary oxidation reactor and wherein the catalyst system comprises cobalt ranging from 500 ppm by weight to 6000 ppm by weight with respect to the weight of the liquid in the reaction medium, manganese in an amount ranging from 2 ppm by weight to 600 ppm by weight with respect to the weight of the liquid in the reaction medium and bromine in an amount ranging from 300 ppm by weight to 4500 ppm by weight with respect to the weight of the liquid in the reaction medium.

In another embodiment of the invention, when the oxidizable raw material stream **30** comprises 5-HMF, then the cobalt to manganese ratio by weight is at least 10:1, 15:1, 20:1, 25:1, 30:1, 40:1, 50:1, 60:1, or 400 to 1.

In another embodiment, when the oxidizable material stream **30** comprises at least one oxidizable compound selected from the group consisting of 5-HMF esters (5-R(CO)OCH₂-furfural where R = alkyl, cycloalkyl and aryl), 5-HMF ethers (5-R'OCH₂-furfural, where R' = alkyl, cycloalkyl and aryl), 5-alkyl furfurals (5-R"-furfural, where R" = alkyl, cycloalkyl and aryl), mixed feedstocks of 5-HMF and 5-HMF esters, mixed feedstocks of 5-HMF and 5-HMF ethers, and mixed feed-stocks of 5-HMF and 5-alkyl furfurals, the cobalt to manganese ratio by weight of the catalyst system is at least 1:1, 10:1, 20:1, 50:1, 100:1, or 400:1.

In one embodiment not belonging to the invention, furan-2,5-dicarboxylic acid (FDCA) can be obtained by liquid phase oxidation of 5-(hydroxymethyl)furfural (5-HMF), 5-(acetoxymethyl)furfural (5-AMF) and 5-(ethoxymethyl)furfural (5-EMF) with molecular oxygen using Co/Mn/Br catalyst system in acetic acid solvent. After the oxidation of 5-HMF/5-AMF/5-EMF in presence of acetic acid, the FDCA precipitates out of solution. After filtration, washing with acetic acid and then with water, and drying, solids were obtained with a minimum of 90%, 92%, 94%, 96% FDCA content by weight.

In another embodiment not belonging to the invention, FDCA is obtained by liquid phase oxidation of 5-HMF, 5-AMF and 5-EMF with molecular oxygen using Co/Mn/Br catalyst system in acetic acid solvent. After the oxidation of 5-HMF/5-AMF/5-EMF in acetic acid, the FDCA precipitates out of solution. After filtration, washing with acetic acid and then with water, and drying, solids were obtained with a minimum of 96% FDCA content and a maximum b* of 15, 16, 17, 18, 19, or 20.

The b* is one of the three-color attributes measured on a spectroscopic reflectance-based instrument. The color can be measured by any device known in the art. A Hunter Ultrascan XE instrument is typically the measuring device. Positive readings signify the degree of yellow (or absorbance of blue), while negative readings signify the degree of blue (or absorbance of yellow).

In another embodiment of the invention, a process is provided for producing furan-2,5-dicarboxylic acid (FDCA) in minimum yields of 80% or 85% or 90% or greater by liquid phase oxidation that minimizes solvent and starting material loss through carbon burn. As used herein, yield is defined as mass of FDCA obtained divided by the theoretical amount of FDCA that should be produced based on the amount of raw material use. For example, if one mole or 126.11 grams of 5-HMF are oxidized, it would theoretically generate one mole or 156.01 grams of FDCA. If for example, the actual amount of FDCA formed is only 150 grams, the yield for this reaction is calculated to be = (150/156.01) times 100, which equals a yield of 96%. The same calculation applies for oxidation reaction conducted using 5-HMF derivatives or mixed feeds.

In another embodiment, a process is disclosed comprising oxidizing at least one oxidizable compound in an oxidizable raw material stream **30** in the presence of an oxidizing gas stream **10**, solvent stream **20**, and at least one catalyst system in a primary oxidation zone **125;** wherein said oxidizable compound is selected from the group consisting of H(C=O)-R-(C=O)H, HOH2C-R-(C=O)H, 5-(hydroxymethyl)furfural (5-HMF); wherein said solvent stream comprises acetic acid with or without the presence of water; wherein said catalyst system comprises cobalt, manganese and bromine, wherein the weight ratio of cobalt to manganese in the reaction mixture is from about 10 to about 400 and the weight ratio of cobalt to bromine is from about 0.7 to about 3.5. Such a catalyst system with improved Co:Mn and Co:Br ratio can lead to high yield of FDCA (minimum of 90%), decrease in the formation of impurities (measured by b*) causing color in the downstream polymerization process while keeping the amount of CO and CO₂ in the off-gas at a minimum.

The temperature in the primary oxidation zone ranges from 100 °C to 220°C, and can range from 110°C to 160 °C or can range from 105°C to 180°C or 100°C to 200°C, or 100°C to 190°C. One advantage of the disclosed primary oxidation conditions is low carbon burn as illustrated in Table 1. Oxidizer off gas stream **120** is routed to the oxidizer off gas treatment zone 825 to generate an inert gas stream **810**, liquid stream **820** comprising water, and a recovered solvent stream **830** comprising condensed solvent. In one embodiment, at least a portion of recovered solvent stream **830** is routed to wash fed stream **620** and the combined stream is routed to the solid-liquid separation zone **625** for the purpose of washing the solids present in the solid-liquid separation zone **625.** In one embodiment, the inert gas stream **810** can be vented to the atmosphere. In another embodiment, at least a portion of the inert gas stream **810** can be used as an inert gas in the process for inerting vessels and or used for conveying gas for solids in the process.

In another embodiment of the invention, the composition of the liquid phase within the primary oxidizer can have a pH from -4.0 to 1.0 or the feedstock pH is from -1.8 to 1.0, or the feedstock pH is from -1.5 to 1.0. It should be understood that steps (b)- (g) are optional and only one possible embodiment of a process to purified the carboxylic acid composition.

**Step (b)** comprises routing the crude carboxylic composition 110 and fresh solvent stream **220** to a liquid displacement zone **225** to produce a displaced mother liquor stream **230** and low impurity slurry stream **210** comprising FDCA. The displaced mother liquor stream **230** comprises solvent and soluble matter dissolved in the solvent comprising dissolved impurities and dissolved catalyst. In various embodiments of the invention, from 5% to 99%, from 30% to 90%, and most preferably from 50 to 85% of mother liquor present in the carboxylic acid composition **110** is displaced in the liquid displacement zone **225** resulting in dissolved matter comprising impurities present in the displaced mother liquor not going forward in the process. Sufficient fresh solvent is fed to the liquid displacement zone **225** that becomes mixed with solids present resulting in a low impurity slurry stream **210** being pumpable with weight % solids ranging from 1% to 50%, 10% to 40%, and preferably the weight % solids in stream **210** will range from 25% to 38%.

The liquid displacement zone may be a single unit operation or multiple unit operations. In one embodiment of the invention, the liquid displacement zone **225** may be any solid-liquid separation device capable of generating an isolated wet cake from a feed slurry and then mixing the isolated wet cake with fresh solvent in a separate mixing device to generate the low impurity slurry stream **210.** Examples of suitable solid-liquid separation devices include, but are not limited to, a continuous pressure drum filter, solid bowl centrifuges including, but not limited to, decanter and disc stack centrifuges, and batch pressure filters including, but not limited to, candle and leaf filters. The preferred solid-liquid separation device for this application is a continuous pressure drum filter. The solid-liquid separator is operated at temperatures between about 30 degrees C. to about 200 degrees C., preferably 80 degrees C. to about 170.degree. C. The solid-liquid separator in the liquid displacement zone **225** may be operated in continuous or batch mode, although it will be appreciated that for commercial processes, the continuous mode is preferred. Alternatively, a portion of the mother liquor in stream **110** is displaced with fresh liquor stream **220** in a single device to form the low impurity slurry stream **210** without forming an isolated wet cake.

In one embodiment, from 5% to 100% by weight of the displaced mother liquor stream **230** is routed to a purge zone **235** wherein a portion of the impurities present in stream **230** are isolated and exit the process as purge stream **920,** wherein a portion is 5% by weight or greater. Recovered solvent stream **910** comprises solvent and catalyst isolated from stream **230** and is recycled to the process. In one embodiment, recovered solvent stream **910** is recycled to the primary oxidation zone **125** and contains greater than 30% of the catalyst that entered the purge zone **235** in stream **230.** In another embodiment, stream **910** is recycled to the primary oxidation zone **125** and contains greater than 50 weight %, contains greater than 70 weight %, and preferably greater than 90 weight % of the catalyst that enters the purge zone **235** in stream **230** on a continuous or batch basis.

In another embodiment of this invention, a portion up to 100% of the carboxylic acid composition **110** may be routed directly to a secondary oxidation zone **335** without being subjected to the liquid displacement zone **225.** In another embodiment of the invention, up to 100% of the feed to the purge zone **235** may be a mother liquor stream **630** generated in a solid-liquid separation zone **625** which also produces the purified wet cake stream **610.**

In yet another embodiment, up to 100% of the feed to the purge zone **235** may be mother liquor generated in a secondary liquid displacement zone located at some location downstream of the secondary oxidation zone **325.** A secondary liquid displacement zone is not show in Figure 1, and it comprises equipment like that described for the liquid displacement zone **225** located after the primary oxidation zone **125**, and must be located after the secondary oxidation zone **335.**

**Step (c)** comprises oxidizing the low impurity slurry stream **210** in a secondary oxidation zone **335** to form a purified slurry stream **310.** In one embodiment of the invention, the low impurity slurry stream **210** is routed to a secondary oxidation zone **335** where it is heated to between 115 degrees C. and 220 degrees C., and preferably between 120 degrees C. to 200 degrees C. and further oxidized with an oxidizing gas, such as air, fed by line **320** to produce a purified slurry stream **310.** The secondary oxidation zone comprises at least one oxidation reactor vessel. In one embodiment, the secondary oxidation zone can be one or more oxidation vessels. When the carboxylic acid in low impurity slurry stream **210** is FDCA, the secondary oxidation zone is operated at a temperature ranging from 115 degrees C. to 220 degrees C., preferably between 120 degrees C. to 200 degrees C., and stream **210** is further oxidized with an oxidizing gas stream fed by line **320** to produce a purified slurry stream **310.**

Generally, oxidation in the secondary oxidation zone **335** is at a higher temperature than the oxidation in the primary oxidation zone **125** to enhance the impurity removal. In one embodiment, the secondary oxidation zone **335** is operated at 30°C, 20°C, and preferably 10°C higher temperature than the oxidation temperature in the primary oxidation zone **125** to enhance the impurity removal. The secondary oxidation zone **335** can be heated directly with solvent vapor, or steam via stream **320** or indirectly by any means known in the art.

Additional purification of the low impurity slurry stream **210** is accomplished in the secondary oxidation zone by a mechanism involving recrystallization or crystal growth and oxidation of impurities and intermediates including FFCA. One of the functions of the secondary oxidation zone is to convert FFCA to FDCA. FFCA is considered monofunctional relative to a polyester condensation reaction because it contains only one carboxylic acid. FFCA is present in the carboxylic acid composition stream **110** and the low impurity slurry stream **210.** FFCA is generated in the primary oxidation zone **125** because the reaction of 5-HMF to FFCA can be about eight times faster than the reaction of FFCA to the desired di-functional product FDCA. Additional air or molecular oxygen may be fed in stream **320** to the secondary oxidation zone **335** in an amount necessary to oxidize a substantial portion of the partially oxidized products such as FFCA in the stream **210** to the corresponding carboxylic acid FDCA. Generally, at least 70% by weight of the FFCA present in the low impurity slurry stream **210** is converted to FDCA in the secondary oxidation zone **335.** Preferably, at least 80% by weight of the FFCA present in the low impurity slurry stream **210** is converted to FDCA in the secondary oxidation zone **335**, and most preferably, at least 90% by weight of the FFCA present in the low impurity slurry stream **210** is converted to FDCA in the secondary oxidation zone **335.** Significant concentrations of monofunctional molecules like FFCA in the dried, purified FDCA product are particularly detrimental to polymerization processes as they may act as chain terminators during the polyester condensation reaction.

The amount of oxygen fed in the secondary oxidation zone **335** in controlled to limit the burning of organic molecules to CO₂. The amount of oxygen in stream **330** is monitored and used to control the amount of oxygen fed in stream **320.** Another function of the secondary oxidation zone **335** is to dissolve and recrystallize solids present in the low impurity slurry stream **210** fed to the secondary oxidation zone. At least 10% by weight, 25% by weight, 50% by weight, and preferably at least 85% by weight of solid impurities and oxidation by-products in stream **210** feed to the secondary oxidation zone **335** go into solution as the FDCA particles are dissolved and re-crystallized in the secondary oxidation zone **335.** Off gas from the secondary oxidation zone is withdrawn via line **330** and fed to a recovery system where the solvent is removed from the off gas comprising volatile organic compounds (VOCs). VOCs including methyl bromide may be treated, for example by incineration in a catalytic oxidation unit. The purified slurry stream **310** generated in the secondary oxidation zone is routed to the crystallization zone **425.**

**Step (d)** comprises crystallizing the secondary oxidation slurry **310** in a crystallization zone **425** to form a crystallized slurry stream **410.** Generally, the crystallization zone **425** comprises at least one crystallizer. Vapor from the crystallization zone can be condensed in at least one condenser and returned to the crystallization zone **425** or **routed away from crystallization zone 425.** Optionally, the liquid from the condenser or vapor product from the crystallization zone can be recycled, or it can be withdrawn or sent to an energy recovery device. In addition, the crystallizer off gas is removed via line **420** and can be routed to a recovery system where the solvent is removed, and crystallizer off gas comprising VOCs may be treated, for example, by incineration in a catalytic oxidation unit. When the carboxylic acid is FDCA, the purified slurry stream **310** from the secondary oxidation zone **335** is fed to a crystallization zone **425** comprising at least one crystallizer where it is cooled to a temperature between 40.degrees C. to 175 degrees C. to form a crystallized slurry stream **410**, preferably to a temperature between 50 degrees C. to 170 degrees C., and most preferably from 60 degrees C. to 165 degrees C.

The crystallized slurry stream **410** is then routed to a cooling zone **430** to generate a cooled crystallized slurry stream **510.** The cooling of the crystallized slurry stream **410** can be accomplished by any means known in the art. Typically, the cooling zone **430** comprises a flash tank. The temperature of stream **510** can range from 35°C to 160°C, 45°C to 120°C, and preferably from 55°C to 95°C.

In another embodiment, a portion of up to 100% of the secondary oxidation slurry stream **310** is routed directly to the cooling zone **425**, thus the portion is not subjected to a crystallization zone **430.** In yet another embodiment, a portion of up to 100% of the crystallized slurry stream **410** is routed directly to a secondary liquid displacement zone which is not illustrated in Figure 1. Up to 100% of the slurry effluent comprising FDCA from a secondary liquid displacement zone can be routed to the solid-liquid separation zone **625** and or routed directly to the cooling zone **430.** The function of the secondary liquid displacement zone is to displace a portion of solvent in the crystallized slurry stream **410** with fresh solvent and or water wherein a portion must be greater than 5 weight percent. The secondary liquid displacement zone is separate and distinct from the liquid displacement zone **225** located after the primary oxidation zone **125.** The same type of equipment may be used for both the primary and secondary liquid displacement zones. In yet another embodiment, crystallized slurry stream **410** can be routed directly to the solid-liquid separation zone **625** without being first processed in the cooling zone **430.**

**Step (e)** comprises isolating, washing, and dewatering solids present in the cooled, crystallized slurry stream **510** in the solid-liquid separation zone **625.** These functions may be accomplished in a single solid-liquid separation device or multiple solid-liquid separation devices. The solid-liquid separation zone **625** comprises at least one solid-liquid separation device capable of separating solids and liquids, washing solids with a wash solvent stream 620, and reducing the % moisture in the washed solids to less than 30 weight %, less than 25 weight %, less than 20 weight%, less than 15 weight %, and preferably less than 10 weight %.

Equipment suitable for the solid liquid separation zone **625** can typically be comprised of, but not limited to, the following types of devices: centrifuges, cyclones, rotary drum filter, belt filters, pressure leaf filters, candle filters, etc. The preferred solid liquid separation device for the solid liquid separation zone **625** is a rotary pressure drum filter. The temperature of the cooled, crystallized slurry steam **510** which is routed to the solid-liquid separation zone **625** can range from 50°C to 140°C, 70°C to 120°C, and is preferably from 75°C to 95°C. The wash solvent stream **620** comprises a liquid suitable for displacing and washing mother liquor from the solids.

In one embodiment of the invention, a suitable wash solvent comprises acetic acid and water. In another embodiment, a suitable solvent comprises water up to 100% water. The temperature of the wash solvent can range from 20°C to 135°C, 40°C and 110°C, and preferably from 50°C to 90°C. The amount of wash solvent used is defined as the wash ratio and equals the mass of wash divided by the mass of solids on a batch or continuous basis. The wash ratio can range from 0.3 to 5, 0.4 to 4, and preferably from 0.5 to 3.

After solids are washed in the solid liquid separation zone, they are dewatered. Dewatering involves reducing the mass of moisture present with the solids to less than 30% by weight, less than 25% by weight, less than 20% by weight, less than 15% by weight, and most preferably less than 10% by weight resulting in the generation of a purified wet cake stream **610.** In one embodiment, dewatering is accomplished in a filter by passing a gas stream through the solids to displace free liquid after the solids have been washed with a wash solvent. In another embodiment, dewatering is achieved by centrifugal forces in a perforated bowl or solid bowl centrifuge. Stream **630** generated in the solid-liquid separation zone **625** is a mother liquor stream comprising oxidation solvent, catalyst, and some impurities and oxidation byproducts. In one embodiment, a portion of stream **630** is routed to a purge zone **235** and a portion is routed back to the primary oxidation zone **125** wherein a portion is at least 5 weight %. Wash liquor stream **640** is also generated in the solid-liquid separation zone **625** and comprises a portion of the mother liquor present in stream **510** and wash solvent wherein the ratio of mother liquor mass to wash solvent mass is less than 3 and preferably less than 2.

**Step (f)** comprises drying the purified wet cake stream **610** in a drying zone **725** to generate a dry purified carboxylic acid **710** and a vapor stream **720.** In one embodiment, vapor stream **720** comprises wash solvent vapor. In another embodiment, vapor stream **720** comprises oxidation solvent and wash solvent. The drying zone **725** comprises at least one dryer and can be accomplished by any means known in the art that is capable of evaporating at least 10% of the volatiles remaining in the purified wet cake stream **610** to produce the dried, purified carboxylic acid **710** comprising purified FDCA and a vapor stream **720.** For example, indirect contact dryers include, but are not limited to, a rotary steam tube dryer, a Single Shaft Porcupine _{RTM} dryer, and a Bepex Solidaire _{RTM} dryer. Direct contact dryers include, but are not limited to, a fluid bed dryer and drying in a convey line can be used for drying to produce stream **710.** The dried, purified carboxylic acid **710** comprising purified FDCA can be a carboxylic acid composition with less than 8% moisture, preferably less than 5% moisture, and more preferably less than 1% moisture, and even more preferably less than 0.5%, and yet more preferably less than 0.1%. In another embodiment of this invention, if the liquid portion of the purified wet cake stream **610** comprises water and contains less than 0.1 weight % acetic acid, less than 500 ppm wt acetic acid, and preferably less than 200 ppm wt, the stream **610** can be fed directly to a polymerization zone without first being dried.

In one embodiment of the invention, a vacuum system can be utilized to draw vapor stream **720** from the drying zone **725.** If a vacuum system is used in this fashion, the pressure of stream **720** at the dryer outlet can range from 1.01 bar (760 mmHg) to 0.53 bar (400 mmHg), from 1.01 bar (760 mmHg) to 0.8 bar (600 mmHg), from 1.01 bar (760 mmHg) to 0.93 bar (700 mmHg), from 1.01 bar (760 mmHg) to 0.95 bar (720 mmHg), and from 1.01 bar (760 mmHg) to 0.98 bar (740 mmHg) wherein pressure is measured in mmHg above absolute vacuum. The contents of the conduit between solid-liquid separation zone **625** and drying zone **725** utilized to transfer the purified wet cake stream **610** comprises wet cake stream and gas wherein gas is the continuous phase. The pressure at the exit of the solid liquid separation zone **625** can be close to that of the pressure where vapor stream **720** exits the drying zone **725**, wherein close is defined as within 0.14 bar (2 psig), within 0.06 bar (0.8 psig), and preferably within 0.03 bar (0.4 psig).

In an embodiment of the invention, the dried, purified carboxylic acid **710** has a b* less than 9.0. In another embodiment of the invention, the b* color of the dried, purified carboxylic acid **710** is less than 6.0. In another embodiment of the invention, the b* color of the dried, purified carboxylic acid **710** is less than 5.0. In another embodiment of the invention, the b* color of the dried, purified carboxylic acid 7**10** is less than 4.0. In another embodiment of the invention, the b* color of the dried, purified carboxylic acid **710** is less than 3. The b* color is one of the three-color attributes measured on a spectroscopic reflectance-based instrument. A Hunter Ultrascan XE instrument in reflectance mode is typically the measuring device. Positive readings signify the degree of yellow (or absorbance of blue), while negative readings signify the degree of blue (or absorbance of yellow).

It should be appreciated that the process zones previously described can be utilized in any other logical order to produce the dried, purified carboxylic acid **710.** It should also be appreciated that when the process zones are reordered that the process conditions may change. It is also understood that all percent values are weight percents.

**Step (g)** is an optionally step comprising decolorizing the FDCA in this process or an esterified FDCA with a diol stream via hydrogenation. In one embodiment, the diol stream comprises ethylene glycol. In another embodiment, the diol stream comprises isomers of cyclohexane diol, preferably the 1-4 cyclohexane diol isomer. The decolorizing of the FDCA in this process or an esterified FDCA can be accomplished by any means known in the art and is not limited to hydrogenation. However, for example, in one embodiment of the invention, the decolorizing can be accomplished by reacting a carboxylic acid that has undergone esterification treatment, for example with ethylene glycol, with molecular hydrogen in the presence of a hydrogenation catalyst in a reactor zone to produce a decolorized carboxylic acid solution or a decolorized ester product.

For the reactor zone, there are no special limitations in the form or construction thereof, subject to an arrangement that allows supply of hydrogen to effect intimate contact of the carboxylic acid or ester product with the catalyst in the reactor zone. Typically, the hydrogenation catalyst is usually a single Group VIII metal or combination of Group VIII metals. Preferably, the catalyst is selected from a group consisting of palladium, ruthenium, rhodium and combination thereof. The reactor zone comprises a hydrogenation reactor that operates at a temperature and pressure sufficient to hydrogenate a portion of the characteristically yellow compounds to colorless derivatives.

### Examples:

This invention can be further illustrated by the following examples of embodiments thereof, although it will be understood that these examples are included merely for the purposes of illustration and are not intended to limit the scope of the invention unless otherwise specifically indicated.

### Examples Set 1

In Examples 1a-3d, glacial acetic acid and the catalyst components in concentrations described in **Tables 1, 2 and 3** were transferred to a 300 mL titanium autoclave equipped with a high pressure condenser, a baffle and an Isco pump. Cobalt, manganese and ionic bromine were provided as cobalt (II) acetate tetrahydrate, manganese (II) acetate and sodium bromide and/or aqueous hydrobromic acid respectively. The autoclave was pressurized with approximately 50 psig of nitrogen and the homogeneous mixture was heated to the desired temperature in a closed system (i.e., with no gas flow) with stirring. At reaction temperature, an air flow of 1500 sccm was introduced at the bottom of the solution and the reaction pressure was adjusted to the desired pressure. A solution of 5-HMF/5-AMF/5-EMF in acetic acid was fed to the mixture at a rate of 0.833 mL/min via a high pressure Isco pump (this is t=0 for the reaction time). After 30 seconds from the start of substrate feeding, 1.0 g of peracetic acid in 5.0 mL of acetic acid was introduced using a blow-case to start the reaction. The feed was stopped after 1 h and the reaction continued for an additional hour at the same conditions of air flow, temperature and pressure. After the reaction time was completed, the air flow was stopped and the autoclave was cooled to room temperature and depressurized. The heterogeneous mixture was filtered to isolate the crude FDCA. The mass of the filtrate was recorded. The crude FDCA was washed with 60 mL of acetic acid two times and then twice with 100 mL of DI water. The washed crude FDCA was oven dried at 110 °C under vacuum overnight and then weighed. The solid and the filtrate were analyzed by Gas Chromatography using BSTFA derivatization method.

The Off-gas was analyzed for CO and CO₂ by ND-1R (ABB, Advanced Optima) and O₂ by a paramagnetism detection system (Servomex, 1440 Model).

### Analytical

***Gas Chromatographic Method*** Process samples were analyzed using a Shimadzu gas chromatograph Model 2010 (or equivalent) equipped with a split/heated injector (300°C) and a flame ionization detector (300°C). A capillary column (60 meter x 0.32 mm ID) coated with (6% cyanopropylphenyl)-methylpolysiloxane at 1.0 µm film thickness (such as DB-1301 or equivalent) was employed. Helium was used as the carrier gas with an initial column head pressure of 2.06 bar (29.5 psi) and an initial column flow of 3.93 mL/minute while the carrier gas linear velocity of 45 cm/second was maintained constant throughout the entire oven temperature program. The column temperature was programmed as follows: The initial oven temperature was set at 80 °C and was held for 6 minutes, the oven was ramped up to 150 °C at 4°C/minute and was held at 150 °C for 0 minute, the oven was ramped up to 240 °C at 10°C/minute and was held at 240 °C for 5 minutes, then the oven was ramped up to 290 °C at 10°C/minute and was held at 290 °C for 17.5 minutes (the total run time was 60 mins). 1.0-µl of the prepared sample solution was injected with a split ratio of 40:1. EZ-Chrom Elite chromatography data system software was used for data acquisition and data processing. The sample preparation was done by weighing 0.1 g (accurate to 0.1 mg) of sample in a GC vial and adding 200.0 µl ISTD solution (1% by volume of decane in pyridine) and 1000 µl of BSTFA (*N*, *O*-bis(trimethylsilyl) trifluoroacetamide) with 1% TMSCl (trimethylchlorosilane) to the GC vial. The content was heated at 80 °C for 30 minutes to ensure complete derivatization. 1.0-µl of this prepared sample solution was injected for GC analysis.

***Color measurement.*** 1) Assemble the Carver Press die as instructed in the directions---place the die on the base and place the bottom 40 mm cylinder polished side face-up.

2) Place a 40 mm plastic cup (Chemplex Plasticup, 39.7 x 6.4 mm) into the die.

3) Fill the cup with the sample to be analyzed. The exact amount of sample added is not important.

4) Place the top 40 mm cylinder polished side face-down on the sample.

5) Insert the plunger into the die. No "tilt" should be exhibited in the assembled die.

6) Place the die into the Carver Press, making sure that it is near the center of the lower platen. Close the safety door.

7) Raise the die until the upper platen makes contact with the plunger. Apply > 20,000 lbs pressure. Then allow the die to remain under pressure for approximately 3 minutes (exact time not critical).

8) Release the pressure and lower the lower platen holding the die.

9) Disassemble the die and remove the cup. Place the cup into a labeled plastic bag (Nasco Whirl-Pak 4 oz).

10) Using a HunterLab Colorquest XE colorimeter, create the following method (Hunterlab EasyQuest QC software, version 3.6.2 or later)
Mode: RSIN-LAV (**R**eflectance **S**pecular **I**ncluded-**L**arge **A**rea **V**iew)
Measurements:
   CIE L* a* b*
   CIE X Y Z

11) Standardize the instrument as prompted by the software using the light trap accessory and the certified white tile accessory pressed against the reflectance port.

12) Run a green tile standard using the certified white tile and compare the CIE X, Y, and Z values obtained against the certified values of the tile. The values obtained should be ± 0.15 units on each scale of the stated values.

13) Analyze the sample in the bag by pressing it against the reflectance port and obtaining the spectrum and L*, a*, b* values. Obtain duplicate readings and average the values for the report.

### Interpretation of Results:

During the oxidation of 5-HMF to FDCA the alcohol site (ArCH₂OH) was converted into carboxylic acid (ArCOOH) mainly via aldehyde (ArCHO), eq 3. Examples 1a, and 1b (**Table-1**) which use catalyst systems consisting of cobalt, manganese and aqueous hydrobromic acid source produced about 90 % yield of FDCA with > 98 % purity of crude FDCA solid and with a b* of about 6. The crude FDCA solid also contains 5-Formylfuran-2-carboxylic acid (FFCA), only the hydroxylmethyl groups are oxidized to carboxylic acid groups, due to incomplete oxidation.

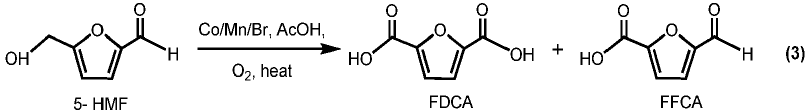

Oxidation of 5-AMF (not belonging to the invention), which contains an oxidizable ester and aldehydes moieties, produced FDCA, FFCA, and acetic acid, eq 4. Examples 2a to 2b (**Table-2**) demonstrate that a minimum of 99% purity FDCA solid with a b* of about 7 or less can be achieved using cobalt, manganese and aqueous hydrobromic acid catalyst system.

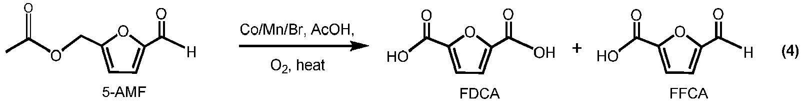

Oxidation of 5-EMF (not belonging to the invention), which contains an oxidizable ether and aldehyde moieties, produced FDCA, FFCA, 5-(ethoxycarbonyl)furan-2-carboxylic acid (EFCA) and acetic acid, eq 5. Examples 3a to 3d (**Table-3**) show that a minimum of 96% purity FDCA solid with a b* of about 6 or less can be achieved using cobalt, manganese and aqueous hydrobromic acid catalyst system.

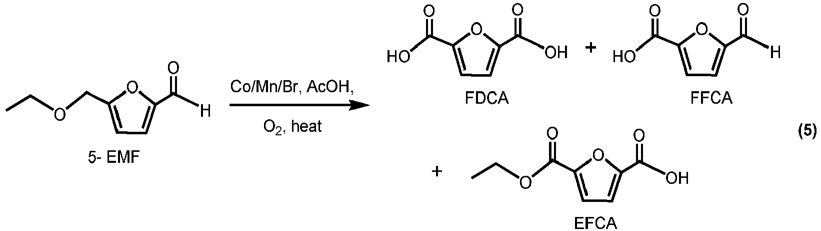

It is very important to note that **in a continuous process** under the same conditions as described in this invention report (which was conducted as a batch process) **even higher purity** of crude FDCA is expected due to efficient mixing, relatively low concentrations of reactive intermediates, and other reasons familiar to those skilled in the art.

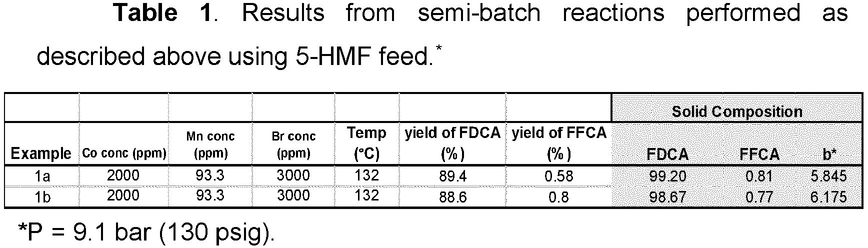

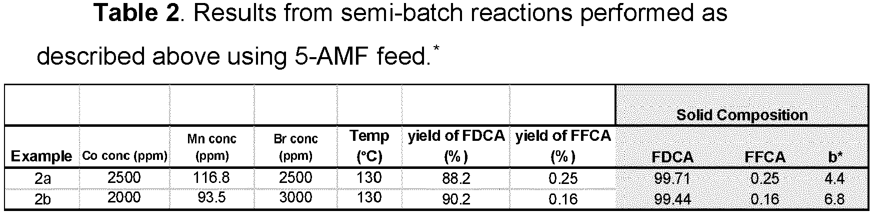

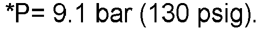

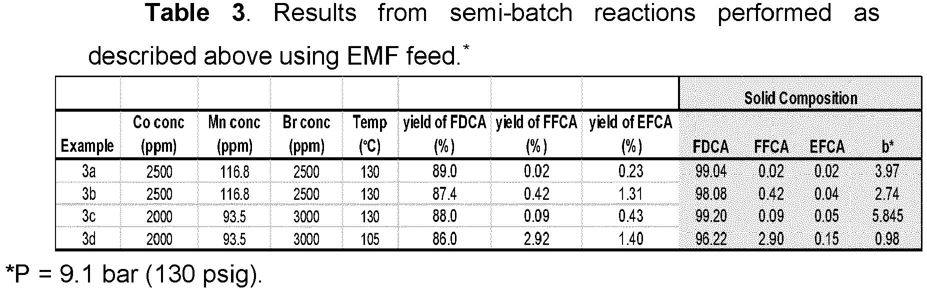

### Examples Set 2

Air oxidation of 5-HMF using cobalt, manganese and ionic bromine catalysts system in acetic acid solvent were conducted. After reaction the heterogeneous mixture was filtered to isolate the crude FDCA. The crude FDCA was washed with acetic acid two times and then twice with DI water. The washed crude FDCA was oven dried at 110 °C under vacuum overnight. The solid and the filtrate were analyzed by Gas Chromatography using BSTFA derivatization method. b* of the solid was measured using a Hunter Ultrascan XE instrument. As shown in **Table 4** we have discovered conditions that to generate yields of FDCA up to 89.4%, b* < 6, and low carbon burn (<0.0006 mol/min CO+CO₂) (Examples 4a and 4b are for comparison).

**Table 4. Results from semi-batch reactions.***

| **Example** | **Bromide source** | **Co conc (ppm)** | **Mn conc (ppm)** | **Br conc (ppm)** | **yield of FDCA (%)** | **yield of FFCA (%)** | **CO (total mol)** | **CO₂ (total mol)** | **CO_{X} (mol/min)** | **pH, before reaction** | **color (b*)** |
|---|---|---|---|---|---|---|---|---|---|---|---|
| 4a | sdid NaBr | 2000 | 93.3 | 3000 | 81.6 | 0.81 | 0.013 | 0.078 | 0.000758 | -0.12 | 13.91 |
| 4b | solid NaBr | 2000 | 93.3 | 3000 | 82.6 | 0.87 | 0.013 | 0.092 | 0.000875 | -0.12 | 14.14 |
| 4c | aqueous HBr | 2000 | 93.3 | 3000 | 89.4 | 0.58 | 0.003 | 0.061 | 0.000533 | -1.03 | 5.845 |
| 4d | aqueous HBr | 2000 | 93.3 | 3000 | 88.6 | 0.8 | 0.0037 | 0.061 | 0.000539 | -1.03 | 6.175 |

| | | | | | | | | | | | |
|---|---|---|---|---|---|---|---|---|---|---|---|
| *P= 9.1 bar (130 psig), COₓ (mol/min) = CO (mol/min) + CO2 (mol/min). | | | | | | | | | | | |

### Examples set 3

In Examples 5a-5h, glacial acetic acid and the catalyst components in concentrations described in **Table-5** were transferred to a 300 mL titanium autoclave equipped with a high pressure condenser, a baffle and an Isco pump. Cobalt, manganese and ionic bromine were provided as cobalt (II) acetate tetrahydrate, manganese (II) acetate and sodium bromide and/or aqueous hydrobromic acid respectively. The autoclave was pressurized with approximately 3.5 bar (50 psig) of nitrogen and then the homogeneous mixture was heated to the desired temperature in a closed system (i.e., with no gas flow) with stirring. At reaction temperature, an air flow of 1500 sccm was introduced at the bottom of the solution and the reaction pressure was adjusted to the desired pressure. A solution of 5-HMF in acetic acid was fed to the mixture at a rate of 0.833 mL/min via a high pressure Isco pump (this is t=0 for the reaction time). After 30 seconds from the start of 5-HMF feeding, 1.0 g of peracetic acid in 5.0 mL of acetic acid was introduced using a blow-case to start the reaction. The feed was stopped after 1 h and the reaction continued for 1 more hour at the same conditions of air flow, temperature and pressure. After the reaction time was completed the air flow was stopped and the autoclave was cooled to room temperature and depressurized. The heterogeneous mixture was filtered to isolate the crude FDCA. The mass of the filtrate was recorded. The crude FDCA was washed with 60 mL of acetic acid two times and then twice with 100 mL of DI water. The washed crude FDCA was oven dried at 110 °C under vacuum overnight and then weighed. The solid and the filtrate were analyzed by Gas Chromatography using BSTFA derivatization method.

The Off-gas was analyzed for CO and CO₂ by ND-1R (ABB, Advanced Optima) and O₂ by a paramagnetism detection system (Servomex, 1440 Model).

### Analytical

***Gas Chromatographic Method*** Process samples were analyzed using a Shimadzu gas chromatograph Model 2010 (or equivalent) equipped with a split/heated injector (300°C) and a flame ionization detector (300°C). A capillary column (60 meter x 0.32 mm ID) coated with (6% cyanopropylphenyl)-methylpolysiloxane at 1.0 µm film thickness (such as DB-1301 or equivalent) was employed. Helium was used as the carrier gas with an initial column head pressure of 29.5 psi and an initial column flow of 3.93 mL/minute while the carrier gas linear velocity of 45 cm/second was maintained constant throughout the entire oven temperature program. The column temperature was programmed as follows: The initial oven temperature was set at 80°C and was held for 6 minutes, the oven was ramped up to 150°C at 4°C/minute and was held at 150 for 0 minute, the oven was ramped up to 240°C at 10°C/minute and was held at 240 for 5 minutes, then the oven was ramped up to 290°C at 10°C/minute and was held at 290 for 17.5 minutes (the total run time was 60 mins). 1.0-µl of the prepared sample solution was injected with a split ratio of 40:1. EZ-Chrom Elite chromatography data system software was used for data acquisition and data processing. The sample preparation was done by weighing 0.1 g (accurate to 0.1 mg) of sample in a GC vial and adding 200.0 µl ISTD solution (1% by volume of decane in pyridine) and 1000 µl of BSTFA (N, O-bis(trimethylsilyl) trifluoroacetamide) with 1% TMSCl (trimethylchlorosilane) to the GC vial. The content was heated at 80°C for 30 minutes to ensure complete derivatization. 1.0-µl of this prepared sample solution was injected for GC analysis.

***pH measurement.*** The electrode for determining non-aqueous pH and millivolts was a Schott N6480-eth series electrode. The LiCl/EtOH filling solution was replaced with (Et)₄N⁺ Br-/ethylene glycol. The electrode response was monitored by Multi-T 2.2 software through a Jensen Systems cdv-70 series Sensolab interface box. The buffers ( pH 4 and 7) were purchased from VWR, and the (Et)₄N⁺ Br/ethylene glycol filling solution was from Metrohm.

To perform the pH measurements, a non-aqueous electrode was initially calibrated using aqueous buffers of 4 and 7 allowing the electrode to equilibrate with each for two to three minutes before calibrating at the respective level. Once the electrode was calibrated within 97.5% of 59.2 millivolts slope, the samples were portioned (~15 mL) into smaller vials with mini-Teflon stir bars. The samples were then placed on a stir plate, and the electrode was lowered into the sample. The depth of the electrode was set to where the sample covered about half of the junction slide. Once the sample and electrode was ready, the sample was measured for non-aqueous pH over a period of three minutes. The time was sufficient for equilibration between the electrode and the sample. Between each sample measurement, the electrode was rinsed with Millipore-grade water and wiped with a Kimwipe. The results were recorded in non-aqueous pH units. The millivolts results were also recorded.

***Color measurement.*** 1) Assemble the Carver Press die as instructed in the directions---place the die on the base and place the bottom 40 mm cylinder polished side face-up.

2) Place a 40 mm plastic cup (Chemplex Plasticup, 39.7 x 6.4 mm) into the die.

3) Fill the cup with the sample to be analyzed. The exact amount of sample added is not important.

4) Place the top 40 mm cylinder polished side face-down on the sample.

5) Insert the plunger into the die. No "tilt" should be exhibited in the assembled die.

6) Place the die into the Carver Press, making sure that it is near the center of the lower platen. Close the safety door.

7) Raise the die until the upper platen makes contact with the plunger. Apply > 20,000 lbs pressure. Then allow the die to remain under pressure for approximately 3 minutes (exact time not critical).

8) Release the pressure and lower the lower platen holding the die.

9) Disassemble the die and remove the cup. Place the cup into a labeled plastic bag (Nasco Whirl-Pak 4 oz).

10) Using a HunterLab Colorquest XE colorimeter, create the following method (Hunterlab EasyQuest QC software, version 3.6.2 or later)
Mode: RSIN-LAV (**R**eflectance **S**pecular **I**ncluded-**L**arge **A**rea **V**iew)
Measurements:
   CIE L* a* b*
   CIE X Y Z

11) Standardize the instrument as prompted by the software using the light trap accessory and the certified white tile accessory pressed against the reflectance port.

12) Run a green tile standard using the certified white tile and compare the CIE X, Y, and Z values obtained against the certified values of the tile. The values obtained should be ± 0.15 units on each scale of the stated values.

13) Analyze the sample in the bag by pressing it against the reflectance port and obtaining the spectrum and L*, a*, b* values. Obtain duplicate readings and average the values for the report.

### Interpretation of Results:

Examples 5c, 5d, 5e and 5f (**Table-5**) which use catalyst systems consisting of cobalt, manganese and aqueous hydrobromic acid or
aqueous hydrobromic acid and sodium bromide as a bromide source produced about 90 % yield of FDCA, minimum colored impurities (measured by b*) and a minimum level of CO and CO₂ (COₓ, mol/min) in the off-gas. One of the reasons for the differences in activity between hydrobromide and sodium bromide, in **a single batch reaction,** is due to faster oxidation of HBr by Mn(III), eq 6, than sodium bromide (it is about 22 times faster: Jiao, X. J.; Espenson, J. H. Inorg. Chem. 2000, 39, 1549). The activity of hydrobromic acid or sodium bromide reaction mediums can be increased by addition of a strong Bronsted acid (such as triflic acid, HCl, etc.).

Mn(OAc)₃ + 2HBr → 2HOAc + Mn(OAc)Br₂ → Mn(OAc)₂ + HBr₂^{•}--------- (**6**)

Comparative examples 5g and 5h show the inhibiting effect of excess manganese. Therefore it is desirable to limit the amount of manganese, during the oxidation process, to achieve high yield of FDCA. Examples 5a and 5b are also comparative.

It is very important to note that **in a continuous process** under the same conditions as described in this invention report (conducted as a batch process) **higher than 90% yield** of FDCA expected due to efficient supply of oxygen and mixing.

**Table 5 Results from semi-batch reactions performed as described above.***

| Example | Bromide source | Co cone (ppm) | Mn cone (ppm) | Brconc (ppm) | yield of FDCA (%) | yield of FFCA (%) | CO (total mol) | CO₂ (total mol) | COx (mol/min) | pH, before reaction | color (b*) |
|---|---|---|---|---|---|---|---|---|---|---|---|
| 5a | solid NaBr | 2000 | 93.3 | 3000 | 81.6 | 0.81 | 0.013 | 0.078 | 0.000758 | -0.12 | 13.91 |
| 5b | solid NaBr | 2000 | 93.3 | 3000 | 82.6 | 0.87 | 0.013 | 0.092 | 0.000875 | -0.12 | 14.14 |
| 5c | aqueous HBr | 2000 | 93.3 | 3000 | 89.4 | 0.58 | 0.003 | 0.061 | 0.000533 | -1.03 | 5.845 |
| 5d | aqueous HBr | 2000 | 93.3 | 3000 | 88.6 | 0.8 | 0.0037 | 0.061 | 0.000539 | -1.03 | 6.175 |
| 5e | aqueous HBr + solid NaBr | 2000 | 93.3 | 3000 | 91.7 | 0.96 | 0.008 | 0.07 | 0000650 | -0.63 | 8.185 |
| 5f | aqueous HBr + solid NaBr | 2000 | 93.3 | 3000 | 90.2 | 0.87 | 0.008 | 0.072 | 0.000667 | -0.63 | 7.95 |
| 5g | aqueous HBr | 2000 | 2000 | 3000 | 79.4 | 1.08 | 0.009 | 0.072 | 0.000675 | -0.84 | 6.21 |
| 5h | aqueous HBr | 2000 | 2000 | 3000 | 80.5 | 1.32 | 0.009 | 0.071 | 0.000667 | -0.84 | 6.31 |

| | | | | | | | | | | | |
|---|---|---|---|---|---|---|---|---|---|---|---|
| * T= 132 C, P = 9.1 bar (P = 130 psig). COₓ (mol/min) = CO (mol/min) + CO₂ (mol/min). FFCA = 5-Formylfuran-2-carboxylic acid. | | | | | | | | | | | |

### Example Set 4

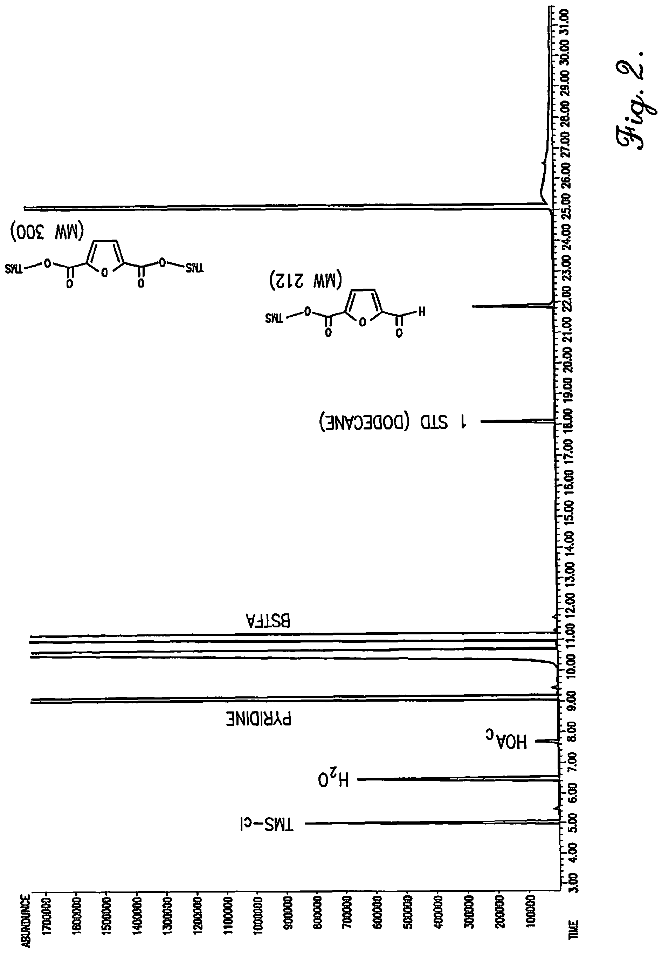

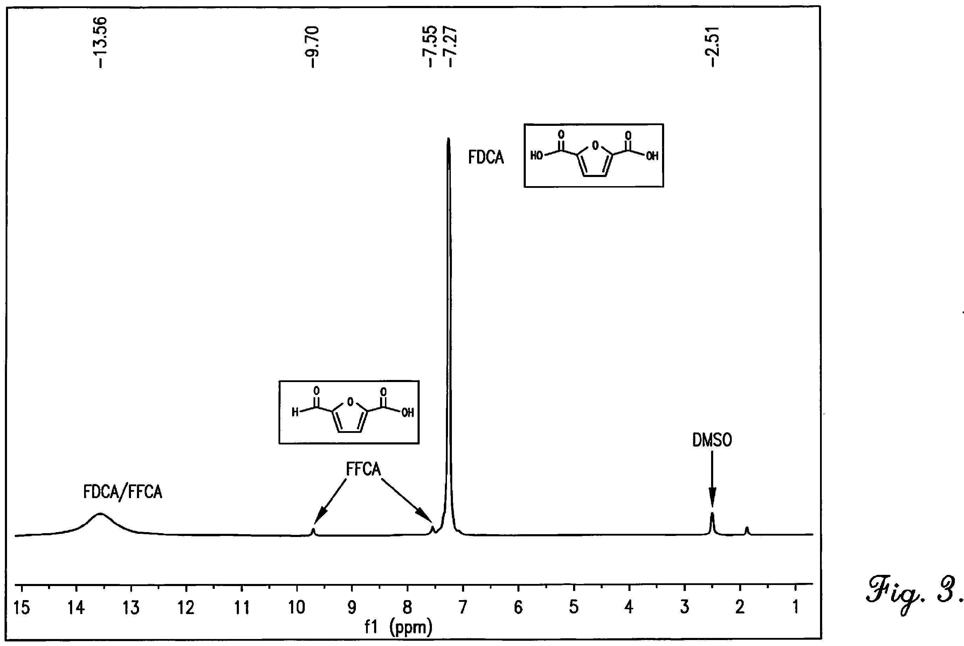

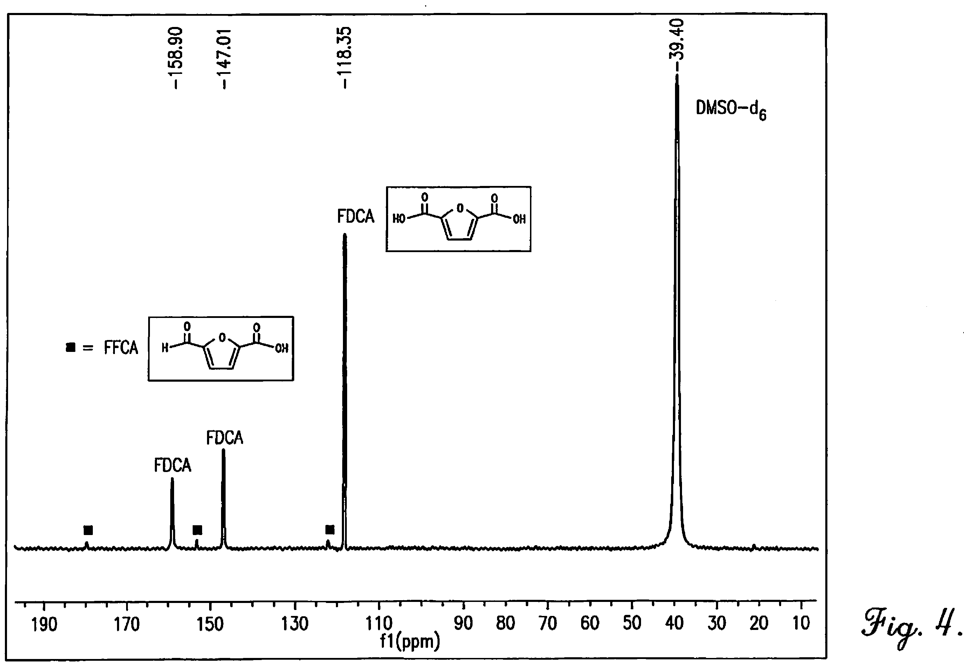

In Examples 1-34, 72, 73, 85, 86, 91, 92 and 93 glacial acetic acid and the catalyst components in concentrations described in the **Tables 6** and **7** were transferred to a 300 mL titanium autoclave equipped with a high pressure condenser, a baffle and an Isco pump. Cobalt, manganese and ionic bromine were provided as cobalt (II) acetate tetrahydrate, manganese (II) acetate and sodium bromide/aqueous hydrobromic acid respectively. The autoclave was pressurized with approximately 3.4 bar (50 psig) of nitrogen and then the homogeneous mixture was heated to the desired temperature in a closed system (i.e., with no gas flow) with stirring. At reaction temperature, an air flow of 1500 sccm was introduced at the bottom of the solution and the reaction pressure was adjusted to the desired pressure. A solution of 5-HMF in acetic acid was fed to the mixture at a rate of 0.833 mL/min via a high pressure Isco pump (this is t=0 for the reaction time). After 30 seconds from the start of 5-HMF feeding, 1.0 g of peracetic acid in 5.0 mL of acetic acid was introduced using a blow-case to start the reaction. The feed was stopped after 1 h and the reaction continued for 1 more hour at the same conditions of air flow, temperature and pressure. After the reaction time was completed the air flow was stopped and the autoclave was cooled to room temperature and depressurized. The heterogeneous mixture was filtered to isolate the crude FDCA. The mass of the filtrate was recorded. The crude FDCA was washed with 60 mL of acetic acid two times and then twice with 100 mL of DI water. The washed crude FDCA was oven dried at 110 °C under vacuum overnight and then weighed. The solid and the filtrate were analyzed by Gas Chromatography using BSTFA derivatization method. A typical GC-chromatogram for isolated crude FDCA sample is shown in **Figure 2****.** The purity of this solid was confirmed by NMR spectroscopy, **Figures 3** and **4****.**

The Off-gas was analyzed for CO and CO₂ by ND-1R (ABB, Advanced Optima) and O₂ by a paramagnetism detection system (Servomex, 1440 Model).

### Analytical

***Gas Chromatographic Method*** Process sample was analyzed using a Shimadzu gas chromatograph Model 2010 (or equivalent) equipped with a split/heated injector (300°C) and a flame ionization detector (300°C). A capillary column (60 meter x 0.32mm ID) coated with (6% cyanopropylphenyl)-methylpolysiloxane at 1.0 µm film thickness (such as DB-1301 or equivalent) was employed. Helium was used as the carrier gas with an initial column head pressure of 29.5 psi and an initial column flow of 3.93 ml/minute while the carrier gas linear velocity of 45 cm/second was maintained constant throughout the entire oven temperature program. The column temperature was programmed as follows: The initial oven temperature was set at 80°C and was held for 6 minutes, the oven was ramped up to 150°C at 4°C/minute and was held at 150 for 0 minute, the oven was ramped up to 240°C at 10°C/minute and was held at 240 for 5 minute, then the oven was ramped up to 290°C at 10°C/minute and was held at 290 for 17.5 minutes (the total run time was 60mins). 1.0-µl of the prepared sample solution was injected with a split ratio of 40:1. EZ-Chrom Elite chromatography data system software was used for data acquisition and data processing. The sample preparation was done by weighing 0.1g (accurate to 0.1mg) of sample in a GC vial and adding 200.0µl ISTD solution (1% by volume of decane in pyridine) and 1000 µl of BSTFA (N, O-bis(trimethylsilyl) trifluoroacetamide) with 1% TMSCl (trimethylchlorosilane) to the GC vial. The content was heated at 80°C for 30 minutes to ensure complete derivatization. 1.0-µl of this prepared sample solution was injected for GC analysis.

### Interpretation of Results:

Experiments 1-34, **Table 6**, were conducted by varying temperature, pressure and levels of cobalt and bromine concentrations to determine optimum conditions and catalyst compositions that produces very high yield of FDCA with minimum amount of carbon burn. In this invention, the weight ratio of cobalt to manganese was deliberately kept very high (i.e. 21) in all the reactions to avoid the inhibiting effect of excess manganese especially below 160 °C.

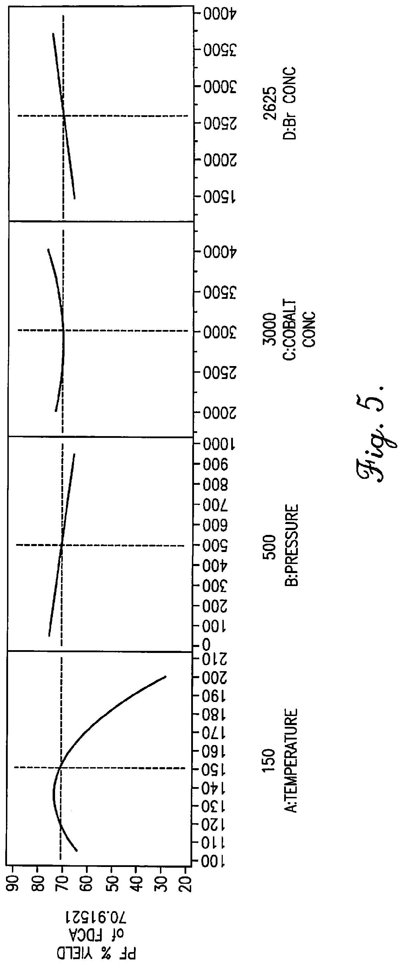

As can be seen from **Figure 5**, under the reaction conditions investigated in this invention the factor that has the most impact on the yield of FDCA is temperature. It is also important to note that the yield increases with increasing cobalt and bromine concentrations.

The data presented in **Table 6** was used to develop a theoretical polynomial model to predict FDCA yield under different conditions, eq - 7. Examples of predicted FDCA yields using this model is given in **Table 7.** Experiments No 72, 73, 85, and 86, **Table 7**, were conducted under the predicted conditions. As can be seen from the results they agree well with the predicted values within experimental error.

Examples 91-93, **Table 8,** were conducted under the US patent applications (US2003/0055271 A1) conditions using our set-up. As can be seen from **Tables 6, 7** and **8**, the patent application conditions gave much inferior yield of FDCA than the current invention.

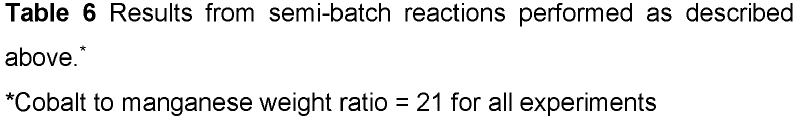

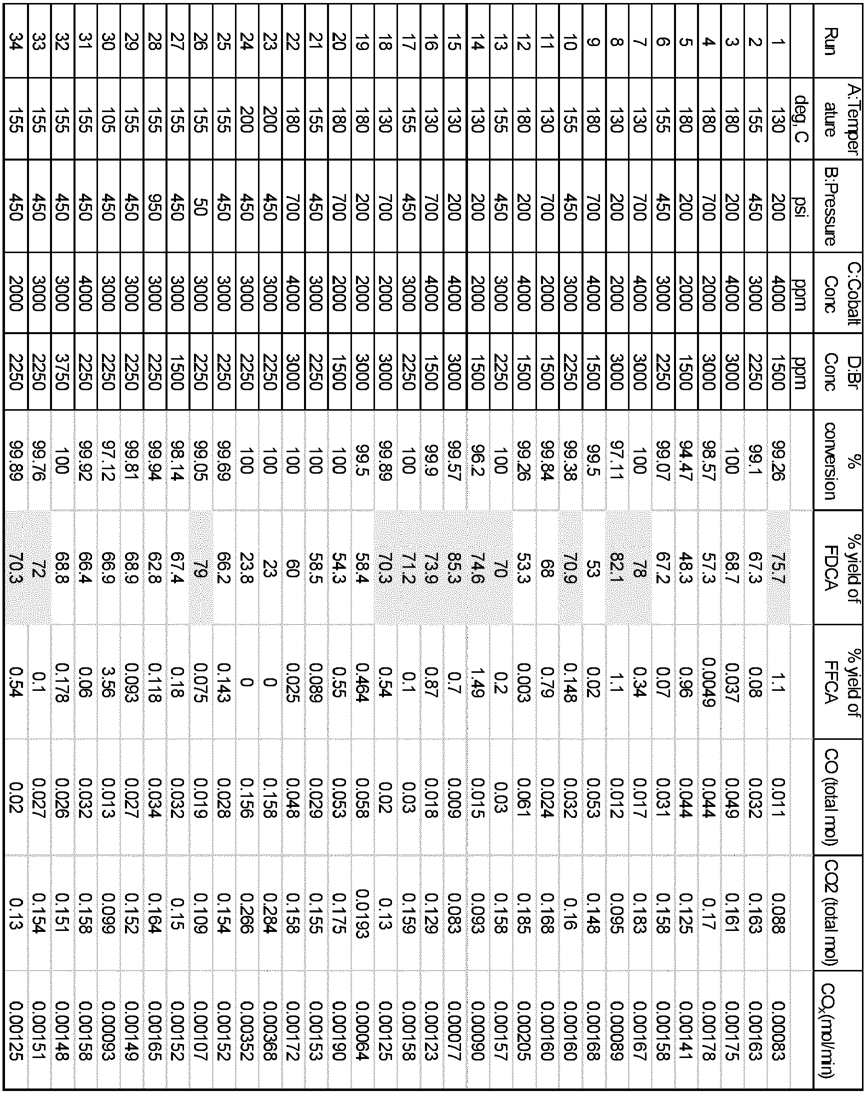

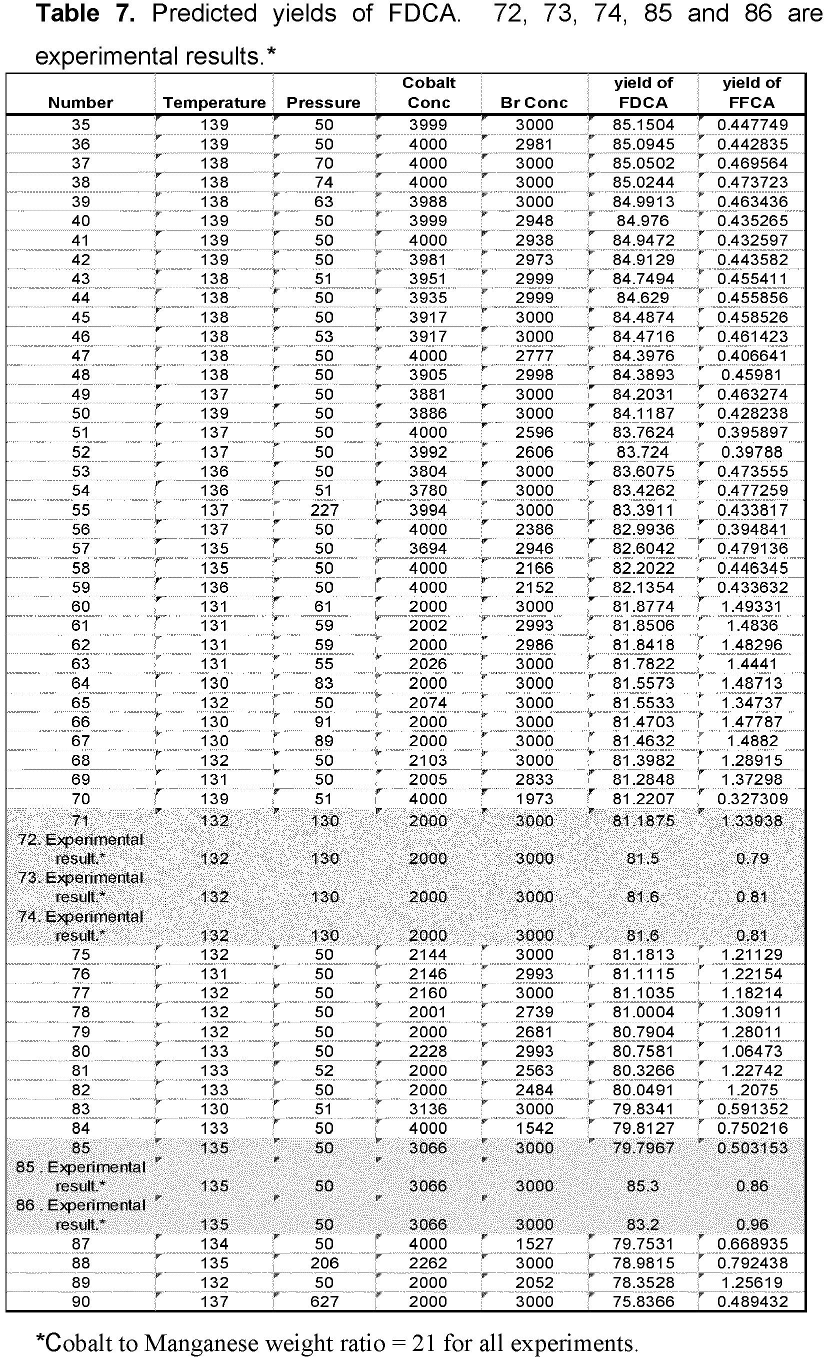

**Table 8. Reactions conducted using the patent application (US20030055271A1) conditions.^{a}**

| Run | A:Terrperature | B:Pressure | C:Cobal Core | D:Br Cone | % conversion | % yield of FDCA | % yield of FFCA | CO (total mol) | CO2 (total mol) | COₓ (mol/rrin) |
|---|---|---|---|---|---|---|---|---|---|---|
| patent (US2003/00552 71A1) | 125 | 950 | 406 | 1102 | | 44.7 | 2.4 | | | |
| 91^{b} | 125 | 950 | 406 | 1102 | 98.42 | 40.1 | 2.3 | 0.03 | 0.128 | 0.00132 |
| patent (US2003/00552 71A1) | 100 | 950 | 406 | 1102 | | 44.8 | 3.3 | | | |
| 92^{b} | 100 | 950 | 406 | 1102 | 60.51 | 0.5 | 4.4 | 0.005 | 0.028 | 0.00028 |
| 93^{b} | 100 | 950 | 406 | 1102 | 64.33 | 0.9 | 2.7 | 0.005 | 0.031 | 0.00030 |

| | | | | | | | | | | |
|---|---|---|---|---|---|---|---|---|---|---|
| ^{a}Cobalt to Manganese ratio = 1. ^{b}Unknown peaks in the GC. | | | | | | | | | | |

### Examples Set 5 (Comparative)

In Examples 9a-11b, glacial acetic acid and the catalyst components in concentrations described in **Tables 9, 10 and 11** were transferred to a 300 mL titanium autoclave equipped with a high pressure condenser, a baffle and an Isco pump. Cobalt, manganese and ionic bromine were provided as cobalt (II) acetate tetrahydrate, manganese (II) acetate and aqueous hydrobromic acid respectively. The autoclave was pressurized with approximately 50 psig of nitrogen and the homogeneous mixture was heated to the desired temperature in a closed system (i.e., with no gas flow) with stirring. At reaction temperature, an air flow of 1500 sccm was introduced at the bottom of the solution and the reaction pressure was adjusted to the desired pressure. A solution of 5-MF/5-AMF/5-EMF in acetic acid was fed to the mixture at a rate of 0.833 mL/min via a high pressure Isco pump (this is t=0 for the reaction time). After 30 seconds from the start of substrate feeding, 1.0 g of peracetic acid in 5.0 mL of acetic acid was introduced using a blow-case to start the reaction. The feed was stopped after 1 h and the reaction continued for an additional hour at the same conditions of air flow, temperature and pressure. After the reaction time was completed, the air flow was stopped and the autoclave was cooled to room temperature and depressurized. The heterogeneous mixture was filtered to isolate the crude FDCA. The mass of the filtrate was recorded. The crude FDCA was washed with 60 mL of acetic acid two times and then twice with 100 mL of DI water. The washed crude FDCA was oven dried at 110 °C under vacuum overnight and then weighed. The solid and the filtrate were analyzed by Gas Chromatography using BSTFA derivatization method.

The off-gas was analyzed for CO and CO₂ by ND-1R (ABB, Advanced Optima) and O₂ by a paramagnetism detection system (Servomex, 1440 Model).

### Analytical

***Gas Chromatographic Method*** Process samples were analyzed using a Shimadzu gas chromatograph Model 2010 (or equivalent) equipped with a split/heated injector (300°C) and a flame ionization detector (300°C). A capillary column (60 meter x 0.32 mm ID) coated with (6% cyanopropylphenyl)-methylpolysiloxane at 1.0 µm film thickness (such as DB-1301 or equivalent) was employed. Helium was used as the carrier gas with an initial column head pressure of 29.5 psi and an initial column flow of 3.93 mL/minute while the carrier gas linear velocity of 45 cm/second was maintained constant throughout the entire oven temperature program. The column temperature was programmed as follows: The initial oven temperature was set at 80 °C and was held for 6 minutes, the oven was ramped up to 150 °C at 4°C/minute and was held at 150 °C for 0 minute, the oven was ramped up to 240 °C at 10°C/minute and was held at 240 °C for 5 minutes, then the oven was ramped up to 290 °C at 10°C/minute and was held at 290 °C for 17.5 minutes (the total run time was 60 mins). 1.0-µl of the prepared sample solution was injected with a split ratio of 40:1. EZ-Chrom Elite chromatography data system software was used for data acquisition and data processing. The sample preparation was done by weighing 0.1 g (accurate to 0.1 mg) of sample in a GC vial and adding 200.0 µl ISTD solution (1% by volume of decane in pyridine) and 1000 µl of BSTFA (N, O-bis(trimethylsilyl) trifluoroacetamide) with 1% TMSCl (trimethylchlorosilane) to the GC vial. The content was heated at 80 °C for 30 minutes to ensure complete derivatization. 1.0-µl of this prepared sample solution was injected for GC analysis.

### Interpretation of Results:

### 5-AMF feed studies:

Oxidation of 5-AMF, which contains an oxidizable ester and aldehydes moieties, produced FDCA, FFCA, and acetic acid, eq 8. Experiments 9a-9k, **Table 9**, were conducted by varying temperature, and levels of cobalt and bromine concentrations to determine optimum conditions and catalyst compositions that produces very high yield of FDCA with minimum amount of carbon burn. In this invention, the weight ratio of cobalt to manganese was deliberately kept very high (i.e. 21) in all the reactions to avoid the inhibiting effect of excess manganese especially below 160 °C. Further discussion on mechanism of initiation and inhibition by Mn(II) in oxidation can be found in Zakharov, I. V. Kinetics and Catalysis 1998, 39, 485; and Jiao, X. J.; Espenson, J. H. Inorg. Chem. 2000, 39, 1549.

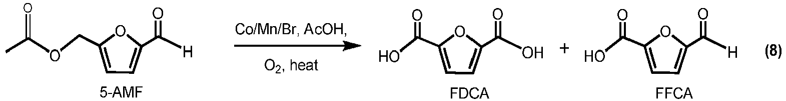

**Table 9. Results from semi-batch reactions performed as described above using 5-AMF feed.***

| | **Factor 1** | **Factor 2** | **Factor 3** | **Factor 4** | **Response 1** | **Response 2** |
|---|---|---|---|---|---|---|
| **Run** | **Temperat ure (°C)** | **Pressure (psig)** | **Cobalt cone (ppmw)** | **Br conc (PPmW)** | **% yield of FDCA** | **% yield of FFCA** |
| 9a | 180 | 130 | 2500 | 2500 | 44.6 | 0.25 |
| 9b | 130 | 130 | 2500 | 2500 | 88.2 | 0.25 |
| 9c | 155 | 130 | 2000 | 3000 | 67.6 | 0.026 |
| 9d | 130 | 130 | 2000 | 3000 | 90.2 | 0.16 |
| 9e | 155 | 130 | 2500 | 2500 | 64.52 | 0.35 |
| 9f | 180 | 130 | 2000 | 3000 | 49.5 | 0.15 |
| 9g | 105 | 130 | 2000 | 3000 | 64.8 | 1.8 |
| 9h | 180 | 130 | 2000 | 3000 | 42.3 | 0.007 |
| 9i | 180 | 130 | 2500 | 2500 | 40.9 | 0.06 |
| 9j | 130 | 130 | 2000 | 3000 | 86.9 | 0.79 |
| 9k | 130 | 130 | 2500 | 2500 | 88.5 | 0.71 |

| | | | | | | |
|---|---|---|---|---|---|---|
| * Cobalt to Manganese weight ratio = 21 for all experiments 130 psig = 9.1 bar | | | | | | |

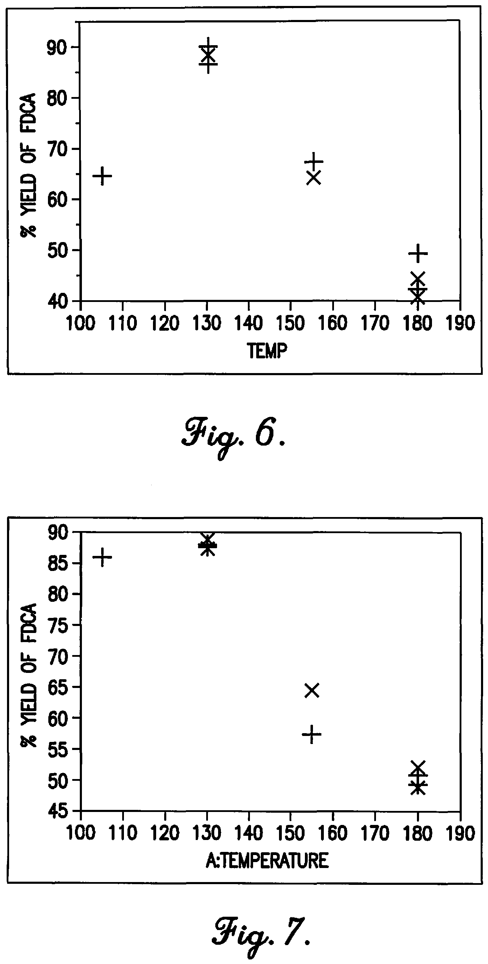

As can be seen from **Figure 6**, under the reaction conditions investigated, in this invention, the factor that has the most impact on the yield of FDCA is temperature. It is also important to note that the yield can increase with increasing cobalt and bromine concentrations.

It is important to note that the same yield and selectivity can be obtained with mixed 5-HMF and 5-AMF feed-stocks with varying ratios of the two components.

### 5-EMF Feed study:

Oxidation of 5-EMF, which contains an oxidizable ether and aldehyde moieties, produced FDCA, FFCA, 5-(ethoxycarbonyl)furan-2-carboxylic acid (EFCA) and acetic acid, eq 9.

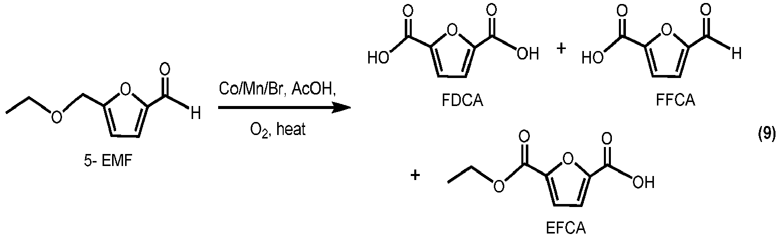

Experiments 10a-10k, **Table 10**, were conducted by varying temperature, and levels of cobalt and bromine concentrations to determine optimum conditions and catalyst compositions that produces very high yield of FDCA with minimum amount of carbon burn. Similar to the 5-AMF oxidation described above the weight ratio of cobalt to manganese was deliberately kept very high (i.e. 21) in all the reactions to avoid the inhibiting effect of excess manganese especially below 160 °C.

**Table 10. Results from semi-batch reactions performed as described above using 5-EMF feed.***

| | **Factor 1** | **Factor 2** | **Factor 3** | **Factor 4** | **Response 1** | **Response 2** | **Response 3** |
|---|---|---|---|---|---|---|---|
| **Run** | **Temperat ure (°C)** | **Pressure (psig)** | **Cobalt conc (ppmw)** | **Br conc (PPmW)** | **% yield of FDCA** | **% yield of FFCA** | **% yield of EFCA** |
| 10a | 180 | 130 | 2500 | 2500 | 52.3 | 0.031 | 0.117 |
| 10b | 130 | 130 | 2500 | 2500 | 88.8 | 0.02 | 0.225 |
| 10c | 155 | 130 | 2000 | 3000 | 57.5 | 0.058 | 0.28 |
| 10d | 130 | 130 | 2000 | 3000 | 87.97 | 0.09 | 0.43 |
| 10e | 155 | 130 | 2500 | 2500 | 64.52 | 0.35 | 0.47 |
| 10f | 180 | 130 | 2000 | 3000 | 49.5 | 0.15 | 0.23 |
| 10g | 105 | 130 | 2000 | 3000 | 86 | 2.92 | 1.4 |
| 10h | 180 | 130 | 2000 | 3000 | 50.9 | 0.096 | 0.24 |
| 10i | 180 | 130 | 2500 | 2500 | 48.9 | 0.4 | 0.61 |
| 10j | 130 | 130 | 2000 | 3000 | 87.5 | 0.4 | 1.22 |
| 10k | 130 | 130 | 2500 | 2500 | 87.4 | 0.42 | 1.3 |

| | | | | | | | |
|---|---|---|---|---|---|---|---|
| * Cobalt to Manganese weight ratio = 21 for all experiments 130psig = 9.1 bar | | | | | | | |

As can be seen from **Figure 7**, under the reaction conditions investigated in this invention the factor that has the most impact on the yield of FDCA is temperature. It is important to note that the same yield and selectivity can be obtained with mixed 5-HMF and 5-EMF feed-stocks with varying ratios of the two components.

### 5-MF Feed study:

Oxidation of 5-MF, which contains an oxidizable methyl and aldehydesmoieties, produced FDCA, and FFCA eq 10. Experiments 11a and 11b, **Table 11**, demonstrate that moderate yield of FDCA with high purity can be obtained using 5-MF as a feed stock.

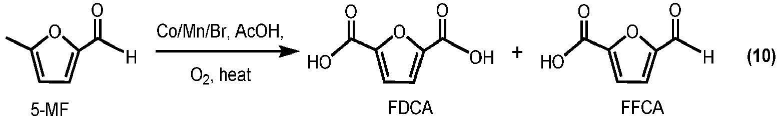

**Table 11. Results from semi-batch reactions performed as described above using 5-MF feed.***

| | **Factor 1** | **Factor 2** | **Factor 3** | **Factor 4** | **Response 1** | **Response 2** | **Response 3** |
|---|---|---|---|---|---|---|---|
| Run | Temperature (°C) | Pressure (psig) | Cobalt Conc (PPmW) | Br Conc (ppmw) | % conversion | % yield of FDCA | % yield of FFCA |
| | | psi | ppm | ppm | | | |
| 11a | 130 | 130 | 2000 | 3000 | 100 | 61 3 | 0.082 |
| 11b | 130 | 400 | 2500 | 2500 | 100 | 61.8 | 0.083 |

| | | | | | | | |
|---|---|---|---|---|---|---|---|
| * Cobalt to Manganese weight ratio = 21. 130 psig = 9.1 bar; 400 psig = 28 bar | | | | | | | |

It is very important to note that **in a continuous process** under the same conditions as described in this invention report (which was conducted as a batch process) with different feed stock **even higher yields** of crude FDCA is expected due to efficient mixing, relatively low concentrations of reactive intermediates, and other reasons familiar to those skilled in the art.

## Claims

1. A carboxylic acid composition comprising:
(a) furan-2,5-dicarboxylic acid in an amount greater than 92 weight percent; and
(b) FFCA in a range of from 0.1 to 4.0 weight percent;
wherein the b* value of the composition is less than 10, the measurement method for determining the b* value being defined in the description;
the carboxylic acid composition being produced by a process comprising:
(a) oxidizing in a primary oxidation zone at least one oxidizable compound in an oxidizable raw material stream comprising 5-HMF in the presence of a solvent stream comprising a saturated organic acid solvent having from 2-6 carbon atoms and a catalyst system at a temperature of 100 °C to 220 °C to produce a carboxylic acid composition; wherein said carboxylic acid composition comprises FDCA; wherein said primary oxidation zone comprises at least one oxidation reactor and wherein said catalyst system comprises cobalt in a range from 500 ppm by weight to 6000 ppm by weight with respect to the weight of the liquid in the primary oxidation zone, manganese in an amount ranging from 2 ppm by weight to 600 ppm by weight with respect to the weight of the liquid in the primary oxidation zone and bromine in an amount ranging from 300 ppm by weight to 4500 ppm by weight with respect to the weight of the liquid in the primary oxidation zone, wherein the source of bromine includes hydrobromic acid; and wherein the oxidation is carried out in the presence of water;
(b) purifying said carboxylic acid composition to produce said dried purified carboxylic acid composition; wherein said purifying comprises a crystallization step, a filtering step and a drying step.

2. A carboxylic acid composition according to claim 1 wherein said carboxylic acid composition has a b* value less than 5.

3. A carboxylic acid composition according to claim 1 where said furan-2,5-dicarboxylic acid is in an amount greater than 94 weight percent.

4. A carboxylic acid composition according to claim 3 wherein said carboxylic acid composition has a b* value less than 10.

5. A carboxylic acid composition according to claim 3 wherein said carboxylic acid composition has a b* value less than 5.

6. A carboxylic acid composition according to claim 1 where said furan-2,5-dicarboxylic acid is in an amount greater than 96 weight percent.

7. A carboxylic acid composition according to claim 6 wherein said carboxylic acid composition has a b* value less than 10.

8. A carboxylic acid composition according to claim 6 wherein said carboxylic acid composition has a b* value less than 5.

9. A carboxylic acid composition according to claim 1 where said furan-2,5-dicarboxylic acid is in an amount greater than 98 weight percent.

10. A composition according to claim 1 wherein said oxidation reactor comprises a bubble column.

11. A composition according to claim 1 wherein said catalyst system comprises cobalt in a range from 700 ppm to 4500 ppm by weight with respect to the weight of the liquid in the primary oxidation zone, manganese in an amount ranging from 20 ppm by weight to 400 ppm by weight with respect to the weight of the liquid in the primary oxidation zone and bromine in an amount ranging from 700 ppm by weight to 4000 ppm by weight with respect to the weight of the liquid in the primary oxidation zone.

## Patentansprüche

1. Eine Carbonsäure-Zusammensetzung aufweisend:
(a) Furan-2,5-dicarbonsäure in einer Menge größer als 92 Gewichtsprozent; und
(b) FFCA in einem Bereich von 0,1 bis 4,0 Gewichtsprozent;
wobei der b* Wert der Zusammensetzung geringer als 10 ist, wobei das Messverfahren zur Bestimmung des b* Werts in der Beschreibung definiert ist;
die Carbonsäure-Zusammensetzung wurde hergestellt durch ein Verfahren aufweisend:
(a) Oxidieren in einem primären Oxidationsbereich von mindestens einer oxidierbaren Verbindung in einem oxidierbaren Rohmaterialstrom aufweisend 5-HMF in Gegenwart von einem Lösungsmittelstrom aufweisend eine gesättigte organische Säure als Lösungsmittel enthaltend von 2 - 6 Kohlenstoffatome und ein Katalysatorsystem bei einer Temperatur von 100 °C bis 220 °C zum Herstellen einer Carbonsäure-Zusammensetzung; wobei besagte Carbonsäure-Zusammensetzung FDCA aufweist; wobei besagter primärer Oxidationsbereich mindestens einen Oxidationsreaktor aufweist und wobei besagtes Katalysatorsystem Cobalt aufweist in einem Bereich von 500 ppm nach Gewicht bis zu 6000 ppm nach Gewicht im Verhältnis zum Gewicht der Flüssigkeit in dem primären Oxidationsbereich, Mangan in einer Menge reichend von 2 ppm nach Gewicht bis 600 ppm nach Gewicht im Verhältnis zu dem Gewicht der Flüssigkeit in dem primären Oxidationsbereich und Brom in einer Menge reichend von 300 ppm nach Gewicht bis 4500 ppm nach Gewicht im Verhältnis zu dem Gewicht der Flüssigkeit in dem primären Oxidationsbereich, und wobei die Quelle des Broms Bromwasserstoffsäure umfasst; und wobei die Oxidation in der Gegenwart von Wasser durchgeführt wird;
(b) Aufreinigen besagter Carbonsäure-Zusammensetzung zum Herstellen besagter trockener, gereinigter Carbonsäure-Zusammensetzung; wobei besagtes Aufreinigen einen Kristallisationsschritt, einen Filtrierschritt und einen Trocknungsschritt aufweist.

2. Carbonsäure-Zusammensetzung nach Anspruch 1, wobei besagte Carbonsäure-Zusammensetzung einen b* Wert geringer als 5 besitzt.

3. Carbonsäure-Zusammensetzung nach Anspruch 1, wobei besagte Furan-2,5-dicarbonsäure in einer Menge größer als 94 Gewichtsprozent ist.

4. Carbonsäure-Zusammensetzung nach Anspruch 3, wobei besagte Carbonsäure-Zusammensetzung einen b* Wert geringer als 10 besitzt.

5. Carbonsäure-Zusammensetzung nach Anspruch 3, wobei besagte Carbonsäure-Zusammensetzung einen b* Wert geringer als 5 besitzt.

6. Carbonsäure-Zusammensetzung nach Anspruch 1, wobei besagte Furan-2,5-dicarbonsäure in einer Menge größer als 96 Gewichtsprozent ist.

7. Carbonsäure-Zusammensetzung nach Anspruch 6, wobei besagte Carbonsäure-Zusammensetzung einen b* Wert geringer als 10 besitzt.

8. Carbonsäure-Zusammensetzung nach Anspruch 6, wobei besagte Carbonsäure-Zusammensetzung einen b* Wert geringer als 5 besitzt.

9. Carbonsäure-Zusammensetzung nach Anspruch 1, wobei besagte Furan-2,5-dicarbonsäure in einer Menge größer als 98 Gewichtsprozent ist.

10. Carbonsäure-Zusammensetzung nach Anspruch 1, wobei besagter Oxidationsreaktor eine Blasenkolonne aufweist.

11. Eine Zusammensetzung nach Anspruch 1, wobei besagtes Katalysatorsystem Cobalt in einem Bereich von 700 ppm bis 4500 ppm nach Gewicht im Verhältnis zu dem Gewicht der Flüssigkeit in dem primären Oxidationsbereich, Mangan in einer Menge reichend von 20 ppm nach Gewicht bis 400 ppm nach Gewicht im Verhältnis zu dem Gewicht der Flüssigkeit in dem primären Oxidationsbereich und Brom in einer Menge reichend von 700 ppm nach Gewicht bis 4000 ppm nach Gewicht im Verhältnis zu dem Gewicht der Flüssigkeit in dem primären Oxidationsbereich aufweist.

## Revendications

1. Composition d'acide carboxylique comprenant :
(a) de l'acide 2,5-furane dicarboxylique en une quantité supérieure à 92 pour cent en poids ; et
(b) du FFCA en une quantité comprise entre 0,1 et 4,0 pour cent en poids ;
dans laquelle la valeur b* de la composition est inférieure à 10, la méthode de mesure pour la détermination de la valeur b* étant définie dans la description ;
la composition d'acide carboxylique étant produite par un procédé comprenant :
(a) l'oxydation dans une zone d'oxydation primaire d'au moins un composé oxydable dans un flux de matières premières oxydables comprenant du 5-HMF en présence d'un flux de solvant comprenant un solvant d'acide organique saturé ayant de 2 à 6 atomes de carbone et un système de catalyseur à une température de 100 °C à 220 °C afin de produire une composition d'acide carboxylique ; dans laquelle ladite composition d'acide carboxylique comprend du FDCA ; dans laquelle ladite zone d'oxydation primaire comprend au moins un réacteur d'oxydation et dans laquelle ledit système de catalyseur comprend du cobalt en une quantité comprise entre 500 ppm en poids et 6000 ppm en poids par rapport au poids du liquide dans la zone d'oxydation primaire, du manganèse en une quantité comprise entre 2 ppm en poids et 600 ppm en poids par rapport au poids du liquide dans la zone d'oxydation primaire et du brome en une quantité comprise entre 300 ppm en poids et 4500 ppm en poids par rapport au poids du liquide dans la zone d'oxydation primaire, dans laquelle la source de brome comprend l'acide bromhydrique ; et dans laquelle l'oxydation est réalisée en présence d'eau ;
(b) la purification de ladite composition d'acide carboxylique afin de produire ladite composition d'acide carboxylique purifié sec; dans laquelle ladite purification comprend une étape de cristallisation, une étape de filtration et une étape de séchage.

2. Composition d'acide carboxylique selon la revendication 1, dans laquelle ladite composition d'acide carboxylique a une valeur b* inférieure à 5.

3. Composition d'acide carboxylique selon la revendication 1 où ledit acide 2,5-furane dicarboxylique est présent en une quantité supérieure à 94 pour cent en poids.

4. Composition d'acide carboxylique selon la revendication 3, dans laquelle ladite composition d'acide carboxylique a une valeur b* inférieure à 10.

5. Composition d'acide carboxylique selon la revendication 3, dans laquelle ladite composition d'acide carboxylique a une valeur b* inférieure à 5.

6. Composition d'acide carboxylique selon la revendication 1 où ledit acide 2,5-furane dicarboxylique est présent en une quantité supérieure à 96 pour cent en poids.

7. Composition d'acide carboxylique selon la revendication 6, dans laquelle ladite composition d'acide carboxylique a une valeur b* inférieure à 10.

8. Composition d'acide carboxylique selon la revendication 6, dans laquelle ladite composition d'acide carboxylique a une valeur b* inférieure à 5.

9. Composition d'acide carboxylique selon la revendication 1 où ledit acide 2,5-furane dicarboxylique est présent en une quantité supérieure à 98 pour cent en poids.

10. Composition selon la revendication 1, dans laquelle ledit réacteur d'oxydation comprend une colonne à bulles.

11. Composition selon la revendication 1, dans laquelle ledit système de catalyseur comprend du cobalt en une quantité comprise entre 700 ppm et 4500 ppm en poids par rapport au poids du liquide dans la zone d'oxydation primaire, du manganèse en une quantité comprise entre 20 ppm en poids et 400 ppm en poids par rapport au poids du liquide dans la zone d'oxydation primaire et du brome en une quantité comprise entre 700 ppm en poids et 4000 ppm en poids par rapport au poids du liquide dans la zone d'oxydation primaire.
